# EUROPEAN PATENT APPLICATION

(11) **EP 4 410 827 A1**
(43) Date of publication of application: **07.08.2024**
(21) Application number: 22863579.3
(22) Date of filing: 01.09.2022
(51) Int. Cl.: C07K 14/605, A61K 38/22, A61K 39/395, C07K 14/645

(54) **GLP-1/GIP DUAL-TARGETED POLYPEPTIDE AND FUSION PROTEIN AND APPLICATIONS THEREOF**

(30) Priority: 02.09.2021 CN 202111026970
(71) Applicant: Sunshine Lake Pharma Co., Ltd., Dongguan, Guangdong 523000 (CN)
(72) Inventor: JIANG, Peng, Dongguan, Guangdong 523871 (CN); XIAO, Lin, Dongguan, Guangdong 523871 (CN); ZHOU, Linjun, Dongguan, Guangdong 523871 (CN); HUANG, Limei, Dongguan, Guangdong 523871 (CN); YANG, Min, Dongguan, Guangdong 523871 (CN); JIANG, Yan, Dongguan, Guangdong 523871 (CN); SUN, Ningyuan, Dongguan, Guangdong 523871 (CN); MAO, Jianming, Dongguan, Guangdong 523871 (CN); LI, Yong, Dongguan, Guangdong 523871 (CN); LI, Lijia, Dongguan, Guangdong 523871 (CN); GUO, Linfeng, Dongguan, Guangdong 523871 (CN); LI, Jing, Dongguan, Guangdong 523871 (CN); LI, Wenjia, Dongguan, Guangdong 523871 (CN)
(74) Representative: Hoffmann Eitle
(86) International application number: PCT/CN2022/116513
(87) International publication number: WO 2023/030444

(57) **Abstract**

A GLP-1/GIP dual-targeted polypeptide, containing a first polypeptide having the following amino acid sequence : X₁X₂X₃GT FX₄SDY SX₅X₆X₇X₈ X₉X₁₀X₁₁X₁₂X₁₃ X₁₄FX₁₅X₁₆W LX₁₇X₁₈X₁₉, wherein X₁ is Y or H, X₂ is A or G or S, X₃ is E or Q, X₄ is I or T, X5 is I or K, X₆ is A or Y or L or I, X₇ is M or L, X₈ is D or E, X₉ is K or E, X₁₀ is I or Q or K or E or L, X₁₁ is H or A or R, X₁₂ is A or Q or V, X₁₃ is K or Q or R or H, X₁₄ is D or E or A or L, X₁₅ is V or I, X₁₆ is N or E or D or Q, X₁₇ is L or I or K or V, X₁₈ is A or E or K, X₁₉ is Q or G; X₁~X₁₃ do not satisfy at the same time that X₁ is Y, X₂ is A, X₃ is E, X₄ is I, X₅ is I, X₆ is A, X₇ is M, X₈ is D, X₉ is K, X₁₀ is I, X₁₁ is H, X₁₂ is Q, X₁₃ is Q, X₁₄ is D, X₁₅ is V, X₁₆ is N, X₁₇ is L, X₁₈ is A, X₁₉ is Q. The GLP-1/GIP dual-targeted polypeptide can effectively reduce the weight and blood sugar level.

## Description

This application claims the priority and benefits of Chinese Patent Application No. 202111026970.5, filed with the State Intellectual Property Office of China on September 02, 2021, which is incorporated herein by reference in its entirety.

### FIELD OF THE INVENTION

The present invention relates to the field of biomedicine, specifically, relates to GLP-1/GIP dual-target polypeptide, fusion protein and applications thereof, more specifically, relates to GLP-1/GIP dual-target polypeptide, nucleic acid molecule encoding GLP-1/GIP dual-target polypeptide, fusion protein, nucleic acid molecule encoding fusion protein, expression vector, recombinant cell, and use of GLP-1/GIP dual-target polypeptide and its nucleic acid molecule, fusion protein and its nucleic acid molecule, expression carrier and recombinant cell in the manufacture of a medicament, pharmaceutical composition and method for preparing fusion protein.

### BACKGROUND ART

Type II diabetes is a chronic metabolic disorder that is closely related to obesity, hyperlipidemia and hypertension. At present, the main effect of drugs for the first-line therapy of type II diabetes is to reduce blood glucose, but the effect of reducing weight is very limited. Glucagon-like peptide-1 (GLP-1) is a polypeptide hormone secreted by the intestinal L-cells after eating, which can stimulate the pancreatic β-cells to secrete insulin, thereby stabilizing the fluctuation of blood glucose after a meal; its effect of lowering blood glucose is glucose concentration dependent, while regulating blood glucose, it greatly reduces the risk of hypoglycemia. In recent years, GLP-1 based drugs, such as liraglutide, duraglutide and semaglutide, have gradually occupied a very important position in diabetes drugs. The GLP-1 drugs also have a weight loss effect when lowering blood glucose, the mechanism is that GLP-1 acts on the gastrointestinal tract to delay gastric emptying and intestinal peristalsis, and acts on the central nervous system to suppress appetite, so as to achieve the purpose of reducing food intake. However, GLP-1 receptor agonist drugs are generally used at larger doses for weight loss, are prone to gastrointestinal side effects, are poorly tolerated, and have a narrow therapeutic window.

Glucose-dependent incretin (GIP) is a peptide hormone secreted by intestinal K cells that contains 42 amino acids and mainly plays a role in glucose homeostasis, protecting pancreatic β cells. GIP and GLP-1 are both incretins, both of which can promote the secretion of insulin and lower blood glucose in a blood glucose concentration-dependent manner, and the GIP-mediated blood glucose lowering effect is even stronger than that of GLP-1. However, possibly due to receptor tolerance induced by hyperglycemia, patients with diabetes have shown insensitivity to GIP, so the use of GIP receptor agonists alone in patients with diabetes do not achieve the goal of improving blood glucose

At present, for diseases with complex mechanisms, such as diabetes, obesity, etc., the effect of single-target drugs is limited, and some traditional treatments, such as insulin injection, oral sulfonylurea and metformin, can easily lead to hypoglycemia, so they still cannot meet the clinical needs. By acting on different targets, multiple effects can be achieved to improve the efficacy of drugs. Therefore, single-molecule GLP-1/GIP dual receptor agonists can effectively solve the shortcomings of existing single-target drugs, and achieve better hypoglycemic and weight-loss effects by simultaneously activating multiple metabolism-related targets, and have great development potential.

### SUMMARY

The present application is based on the inventors' discovery and knowledge of:

Single-molecule GLP-1/GIP dual receptor agonists can effectively solve the shortcomings of existing single-target drugs, the inventors mutated wild type GLP-1 and GIP through a large number of experiments, and obtained a variety of GLP-1/GIP dual-target polypeptides that can simultaneously recognize GLP-1 receptor and GIP receptor, the GLP-1/GIP dual-target polypeptide was fused with the Fc fragment of immunoglobulin to obtain the fusion protein by using a linker peptide. The inventors found that the fusion protein has a good ability to simultaneously activate the GLP-1 receptor and the GIP receptor, and different mutations will increase or decrease the agonistic activity of the GLP-1 receptor and the GIP receptor.

Therefore, in the first aspect, the present invention provides a GLP-1/GIP dual-target polypeptide. According to the embodiments of the present invention, the dual-target polypeptide comprises a first polypeptide, and the first polypeptide has the following amino acid sequence: X₁X₂X₃GTFX₄SDYSX₅X₆X₇X₈ X₉X₁₀X₁₁X₁₂X₁₃ X₁₄FX₁₅X₁₆WLX₁₇X₁₈X₁₉, wherein, X₁ is Y or H, X₂ is A, G or S, X₃ is E or Q, X₄ is I or T, X5 is I or K, X₆ is A, Y, L or I, X₇ is M or L, X₈ is D or E, X₉ is K or E, X₁₀ is I, Q, K, E or L, X₁₁ is H, A or R, X₁₂ is A, Q or V, X₁₃ is K, Q, R or H, X₁₄ is D, E, A or L, X₁₅ is V or I, X₁₆ is N, E, D or Q, X₁₇ is L, I, K or V, X₁₈ is A, E or K, X₁₉ is Q or G; X₁~X₁₃ do not simultaneously satisfy that X₁ is Y, X₂ is A, X₃ is E, X₄ is I, X₅ is I, X₆ is A, X₇ is M, X₈ is D, X₉ is K, X₁₀ is I, X₁₁ is H, X₁₂ is Q, X₁₃ is Q, X₁₄ is D, X₁₅ is V, X₁₆ is N, X₁₇ is L, X₁₈ is A, X₁₉ is Q. The inventors carried out site-directed mutation of wild type GLP-1 and GIP, and the obtained GLP-1/GIP dual-target polypeptide has certain binding activity with GLP-1 and/or GIP receptors.

According to the embodiments of the present invention, the GLP-1/GIP dual-target polypeptide may further comprise at least one of the following additional technical features:

According to the embodiments of the present invention, the dual-target polypeptide comprises a first polypeptide, and the first polypeptide has the following amino acid sequence: X₁X₂EGTFTSDYSIX₆LDKX₁₀AQX₁₃X₁₄FX₁₅X₁₆WLX₁₇AX₁₉, wherein, X₁ is Y or H; X₂ is A, G or S; X₆ is A, Yor L; X₁₀ is I, Q, K or L; X₁₃ is Q or R; X₁₄ is D, E or A; X₁₅ is V or I; X₁₆ is E, D or Q; X₁₇ is L, I or K; X₁₉ is Q or G; wherein, the first polypeptide does not comprise the amino acid sequence of YAEGTFISDYSIAMDKIHQQDFVNWLLAQ (SEQ IDNO:122). The inventors carried out site-directed mutation of wild type GLP-1 and GIP, and the obtained GLP-1/GIP dual-target polypeptide has good biological activity of binding with GLP-1 and/or GIP receptors, thus, the GLP-1 receptor and GIP receptor can be activated simultaneously, and the GLP-1/GIP dual-target polypeptide has the dual functions of weight loss and hypoglycemia.

According to the embodiments of the present invention, the GLP-1/GIP dual-target polypeptide further comprises a second polypeptide, and the second polypeptide has an amino acid sequence shown in SEQ ID NO:1. The specific amino acid sequence of SEQ ID NO:1 is: GPSSGAPPPS.

According to the embodiments of the present invention, the C-terminal amino acid of the first polypeptide is connected with the N-terminal amino acid of the second polypeptide.

According to the embodiments of the present invention, the GLP-1/GIP dual-target polypeptide has at least one of the amino acid sequences shown in Table 1.

In the second aspect, the present invention provides a fusion protein. According to the embodiments of the present invention, the fusion protein comprises: (1) the GLP-1/GIP dual-target polypeptide as described in the first aspect; (2) an Fc fragment, and the C-terminus of the GLP-1/GIP dual-target polypeptide is connected with the N-terminus of the Fc fragment. The fusion protein according to the embodiments of the present invention also has excellent binding activity with GLP-1 and/or GIP receptors, has the dual functions of weight loss and hypoglycemia, and can effectively control or reduce body weight and blood glucose levels.

According to the embodiments of the present invention, the fusion protein may further comprise at least one of the following additional technical features:

According to the embodiments of the present invention, the Fc fragment is an Fc fragment of a human IgG4 or variant thereof.

According to the embodiments of the present invention, the Fc fragment comprises an amino acid sequence shown in SEQ ID NO: 113.

GluSerLysTyrGlyProProCysProProCysProAlaProGluAlaAlaGlyGlyProSerValPheL euPheProProLysProLysAspThrLeuMetIle SerArgThrProGluValThrCysVal Val ValAspVal SerGlnGl uAspProGluValGlnPheAsnTrpTyrValAspGlyValGluValHisAsnAlaLysThrLysProArgGluGluGlnP heAsnSerThrTyrArgValValSerValLeuThrValLeuHisGlnAspTrpLeuAsnGlyLysGluTyrLysCysLys ValSerAsnLysGlyLeuProSerSerIleGluLysThrIleSerLysAlaLysGlyGlnProArgGluProGlnValTyrTh rLeuProProSerGlnGluGluMetThrLysAsnGlnValSerLeuThrCysLeuValLysGlyPheTyrProSerAspIl eAlaValGluTrpGluSerAsnGlyGlnProGluAsnAsnTyrLysThrThrProProValLeuAspSerAspGlySerP hePheLeuTyrSerArgLeuThrValAspLysSerArgTrpGlnGluGlyAsnValPheSerCysSerValMetHisGlu AlaLeuHisAsnHisTyrThrGlnLysSerLeuSerLeuSerLeuGly (SEQ ID NO: 113).

According to the embodiments of the present invention, the fusion protein further comprises a linker peptide with an amino acid sequence shown in SEQ ID NO: 114.

GlyGlyGlyGlySerGlyGlyGlyGlySerGlyGlyGlyGlySerAla (SEQ ID NO: 114).

According to the embodiments of the present invention, the N-terminus of the linker peptide is connected with the C-terminus of the GLP-1/GIP dual-target polypeptide, and the C-terminus of the linker peptide is connected with the N-terminus of the Fc fragment.

In the third aspect, the present invention provides a nucleic acid molecule. According to the embodiments of the present invention, the nucleic acid molecule encodes the GLP-1/GIP double-target polypeptide of the first aspect or the fusion protein of the second aspect. According to the embodiments of the present invention, the GLP-1/GIP dual-target polypeptide or fusion protein encoded by the nucleic acid molecule both have excellent binding activity with GLP-1 and/or GIP receptor, and have dual functions of weight loss and hypoglycemia, and can effectively control or reduce body weight and blood glucose levels.

In the fourth aspect, the invention provides an expression vector. According to the embodiments of the present invention, the expression vector comprises the nucleic acid molecule described in the third aspect.

According to the embodiments of the present invention, the expression vector may further comprise at least one of the following additional technical features:

According to the embodiments of the present invention, the expression vector is a eukaryotic expression vector.

In the fifth aspect, the present invention provides a recombinant cell. According to the embodiments of the present invention, the recombinant cell carries the nucleic acid molecule described in the third aspect or the expression vector described in the fourth aspect. The recombinant cells according to the embodiments of the present invention can express the GLP-1/GIP dual-target polypeptide or fusion protein, and the GLP-1/GIP dual-target polypeptide and fusion protein have excellent binding activity with GLP-1 and/or GIP receptors, and have dual functions of weight loss and hypoglycemia, and can effectively control or reduce body weight and blood glucose levels.

In the sixth aspect, the invention provides a pharmaceutical composition. According to the embodiments of the present invention, the pharmaceutical composition comprises the GLP-1/GIP dual-target polypeptide described in the first aspect, the fusion protein described in the second aspect, the nucleic acid molecule described in the third aspect, the expression vector described in the fourth aspect, or the recombinant cell described in the fifth aspect. The pharmaceutical composition may comprise a pharmaceutically acceptable adjuvant, and the pharmaceutically acceptable adjuvant comprises at least one of stabilizers, wetting agents, emulsifiers, binders, isotonic agents; the pharmaceutical composition is at least one kind of tablets, granules, powders, capsules, solutions, suspensions or lyophilized preparations. According to the embodiments of the present invention, the pharmaceutical composition has a long-term function of weight loss and/or hypoglycemia, and can effectively control or reduce body weight and blood glucose levels.

In the seventh aspect, the present invention provides use of the GLP-1/GIP dual-target polypeptide described in the first aspect, the fusion protein described in the second aspect, the nucleic acid molecule described in the third aspect, the expression vector described in the fourth aspect, or the recombinant cell described in the fifth aspect in the manufacture of a medicament. According to the embodiments of the present invention, the medicament is used for controlling or lowering glucose and body weight.

In the eighth aspect, the present invention provides a method for preparing the fusion protein described in the second aspect. According to the embodiments of the present invention, the method comprises: 1) constructing the expression vector described in the fourth aspect; 2) introducing the expression vector into host cells to obtain recombinant cells to express the fusion protein. The fusion protein prepared by the method provided therein has excellent binding activity with GLP-1 and/or GIP receptors, has the dual functions of weight loss and hypoglycemia, and can effectively control or reduce body weight and blood glucose levels.

In the ninth aspect, the invention provides a method for lowering blood glucose and/or body weight of patients. According to the embodiments of the present invention, the method comprises administering to the patients at least one of the following: 1) a GLP-1/GIP dual-target polypeptide as described in the first aspect; 2) a fusion protein as described in the second aspect; 3) a nucleic acid molecule as described in the third aspect; 4) an expression vector as described in the fourth aspect; 5) a recombinant cell as described in the fifth aspect; and 6) a pharmaceutical composition as described in the sixth aspect. The method provided therein can effectively and long-term control or reduce the body weight and/or blood glucose levels of patients.

Additional aspects and advantages of the invention will be set forth in part in the description which follows, and in part will be obvious from the description, or may be learned by practice of the invention.

### DESCRIPTION OF THE DRAWINGS

The above and/or additional aspects and advantages of the present invention will become apparent and readily understood from the description of the embodiments in conjunction with the following figures, wherein:
Figure 1 shows the blood glucose values of HEC-G123 group, HEC-G128 group, HEC-G131 group, HEC-G132 group at different times of the embodiments of the present invention;
Figure 2 shows the area under the curve of HEC-G123 group, HEC-G128 group, HEC-G131 group, HEC-G132 group of the embodiments of the present invention;
Figure 3 shows the blood glucose values of HEC-G113 group, HEC-G122 group, HEC-G126 group, HEC-G127 group at different time of the embodiments of the present invention;
Figure 4 shows the area under the curve of HEC-G113 group, HEC-G122 group, HEC-G126 group, HEC-G127 group of the embodiments of the present invention;
Figure 5 shows the in vivo hypoglycemic effect of HEC-G20 in a db/db mouse model of the embodiments of the present invention;
Figure 6 shows the glycosylated hemoglobin value of HEC-G20 in db/db mouse model of the embodiments of the present invention;
Figure 7 shows the body weight effect of long-term repeated administration of HEC-G115 and HEC-G124 in the embodiments of the present invention of obese mice in a DIO model;
Figure 8 shows the cumulative intake effect of long-term repeated administration of HEC-G115 and HEC-G124 in the embodiments of the present invention of obese mice in a DIO model;
Figure 9 shows the structural diagram of the fusion protein of the embodiments of the present invention, wherein the fusion protein comprises three domains, from N-terminal to C-terminus: GLP-1/GIP dual receptor agonist (GLP-1/GIP dual-target polypeptide), linker peptide and Fc fragment.GLP-1/GIP dual receptor agonist polypeptide.

### EXAMPLES

Embodiments of the invention are described in detail below, examples of which are illustrated in the accompanying drawings. The embodiments described below with reference to the drawings are exemplary and are intended to explain the present invention and are not to be construed as limiting the present invention.

In addition, the terms "first" and "second" are used for descriptive purposes only and cannot be understood as indicating or implying relative importance or implicitly indicating the quantity of indicated technical features. Therefore, features defined as "first" and "second" may explicitly or implicitly include at least one of these features. In the description of the present invention, "plurality" means at least two, such as two, three, etc., unless otherwise expressly and specifically limited.

It should be noted that in this article, the term "receptor agonist" refers to a substance that acts on a receptor and can cause receptor activation, thus causing a biological effect. This effect may be the enhancement or weakening of a specific manifestation of cell activity.

It should be noted that in this article, the terms "GLP-1/GIP dual receptor agonist", "GLP-1/GIP dual-target polypeptide" and "recombinant polypeptide" all refer to polypeptide obtained by site-directed mutation of wild-type GLP-1 and GIP protein molecules, the amino acid sequence of the polypeptide that can simultaneously activate the GLP-1 receptor and the GIP receptor obtained after site-directed mutation of the wild-type GLP-1 and GIP protein molecules is shown in Table 1.

It should be noted that in this article, the term "nucleic acid molecule" can be any polymer containing deoxyribonucleotides or ribonucleotides, including but not limited to modified or unmodified DNA and RNA, and its length does not subject to any special restrictions. For constructs used to construct recombinant cells, it is preferred that the nucleic acid is DNA because DNA is more stable and easier to manipulate than RNA. The "nucleic acid" mentioned in this application actually includes any one or both of the complementary double strands. Those skilled in the art will also understand that one strand can be used to detect another strand and vice versa.

It should be noted that the "construct" described in this application refers to a genetic vector that contains a specific nucleic acid sequence and can transfer the target nucleic acid sequence into host cells to obtain recombinant cells. According to the embodiments of the present invention, the form of the construct is not particularly limited. According to the embodiments of the present invention, the construct may be at least one of plasmid, phage, artificial chromosome, cosmid, and virus, with plasmid being preferred. As a genetic vector, plasmid is simple to operate and can carry larger fragments, and is easy to operate and handle. The form of the plasmid is not particularly limited, it can be either a circular plasmid or a linear plasmid, that is, it can be single-stranded or double-stranded. Those skilled in the art can make selections as needed.

The invention provides a GLP-1/GIP dual-target polypeptide, which comprises a first polypeptide having the following amino acid sequence: X₁X₂X₃GTFX₄SDYSX₅X₆X₇X₈ X₉X₁₀X₁₁X₁₂X₁₃ X₁₄FX₁₅X₁₆WLX₁₇X₁₈X₁₉, wherein, X₁ is Y or H, X₂ is A, G or S, X₃ is E or Q, X₄ is I or T, X5 is I or K, X₆ is A, Y, L or I, X₇ is M or L, X₈ is D or E, X₉ is K or E, X₁₀ is I, Q, K, E or L, X₁₁ is H, A or R, X₁₂ is A, Q or V, X₁₃ is K, Q, R or H, X₁₄ is D, E, A or L, X₁₅ is V or I, X₁₆ is N, E, D or Q, X₁₇ is L, I, K or V, X₁₈ is A, E or K, X₁₉ is Q or G; X₁~X₁₃ do not simultaneously satisfy that X₁ is Y, X₂ is A, X₃ is E, X₄ is I, X₅ is I, X₆ is A, X₇ is M, X₈ is D, X₉ is K, X₁₀ is I, X₁₁ is H, X₁₂ is Q, X₁₃ is Q, X₁₄ is D, X₁₅ is V, X₁₆ is N, X₁₇ is L, X₁₈ is A, X₁₉ is Q. The inventors carried out site-directed mutation of wild-type GLP-1 and GIP, and the obtained GLP-1/GIP dual-target polypeptide has certain binding activity with GLP-1 and/or GIP receptors.

According to a specific embodiment of the present invention, the GLP-1/GIP dual-target polypeptide comprises a first polypeptide, and the first polypeptide has the following amino acid sequence: X₁X₂EGTFTSDYSIX₆LDKX₁₀AQX₁₃X₁₄FX₁₅X₁₆WLX₁₇AX₁₉, wherein, X₁ is Y or H, X₂ is A or G or S, X₆ is A, Y or L, X₁₀ is I, Q, K or L, X₁₃ is Q or R, X₁₄ is D, E or A, X₁₅ is V or I, X₁₆ is E, D or Q, X₁₇ is L , I or K, X₁₉ is Q or G; wherein, the first polypeptide does not comprise the amino acid sequence YAEGTFISDYSIAMDKIHQQDFVNWLLAQ (SEQ IDNO: 122). The inventors carried out site-directed mutation of wild-type GLP-1 and GIP, and the obtained GLP-1/GIP dual-target polypeptide can activate GLP-1 receptor and GIP receptor respectively, the GLP-1/GIP dual-target polypeptide has the dual functions of weight loss and hypoglycemia.

According to a specific embodiment of the present invention, the GLP-1/GIP dual-target polypeptide further comprises a second polypeptide, and the second polypeptide has the amino acid sequence shown in SEQ ID NO: 1. The specific amino acid sequence of SEQ ID NO: 1 is: GPSSGAPPPS.

According to a specific embodiment of the present invention, the C-terminal amino acid of the first polypeptide is connected with the N-terminal amino acid of the second polypeptide.

According to a specific embodiment of the present invention, the GLP-1/GIP dual-target polypeptide has at least one of the amino acid sequences shown in Table 1.

**Table 1:**

| No. | Name of polypeptide | Amino acid sequence of GLP-1/GIP dual-target polypeptide |
|---|---|---|
| 1 | CG01 | YAEGT FTSDY SIYLD KQAAK EFVNW LLAG GPSSGAPPPS (SEQ ID NO:2) |
| 2 | CG02 | YGEGT FTSDY SIYLD KQAAK EFVNW LLAG GPSSGAPPPS (SEQ ID NO:3) |
| 3 | CG03 | YGEGT FTSDY SIALD KQAAK EFVNW LLAG GPSSGAPPPS (SEQ ID NO:4) |
| 6 | CG06 | YGEGT FTSDY SIYLD KKAQR DFVEW LLAQ GPSSGAPPPS (SEQ ID NO:5) |
| 7 | CG07 | YGEGT FTSDY SIALD KIAQK AFVQW LIAG GPSSGAPPPS (SEQ ID NO:6) |
| 8 | CG08 | YGEGT FTSDY SIYLD KIAQKAFVQW LIAG GPSSGAPPPS (SEQ ID NO:7) |
| 9 | CG09 | YGEGT FTSDY SIALD KIAAK AFVQW LIAG GPSSGAPPPS (SEQ ID NO:8) |
| 10 | CG10 | YGEGT FTSDY SIALD KQAAKAFVQW LIAG GPSSGAPPPS (SEQ ID NO:9) |
| 11 | CG11 | YGEGT FTSDY SIALD KQRAK DFVQW LIAG GPSSGAPPPS (SEQ ID NO:10) |
| 13 | CG13 | YSEGT FTSDY SKLLE EEAVR LFIEW LLAG (SEQ ID NO:11) |
| 14 | CG14 | YSEGT FTSDY SKLLE EEAVR LFIEW LVKG (SEQ ID NO:12) |
| 15 | CG20 | YGEGT FTSDY SIYLD KKAQR DFVEW LLAG GPSSGAPPPS (SEQ ID NO:13) |
| 16 | CG21 | YGEGT FTSDY SIYLD KKAQR DFVEW LKAG GPSSGAPPPS (SEQ ID NO:14) |
| 17 | CG22 | YGEGT FTSDY SIYLD KKAQR DFVEW LVKG GPSSGAPPPS (SEQ ID NO:15) |
| 18 | CG23 | YGEGT FTSDY SIYLD KKAQR DFVEW LIAG GPSSGAPPPS (SEQ ID NO:16) |
| 19 | CG24 | YGEGT FTSDY SIALD KKAQR DFVEW LLAQ GPSSGAPPPS (SEQ ID NO:17) |
| 20 | CG25 | YGEGT FTSDY SIALD KKAQR DFIEW LLAQ GPSSGAPPPS (SEQ ID NO:18) |
| 21 | CG26 | YGEGT FISDY SIYLD KKAQR DFVEW LLAQ GPSSGAPPPS (SEQ ID NO:19) |
| 23 | CG28 | YGEGT FTSDY SIYLD KKAQR DFVQW LLAQ GPSSGAPPPS (SEQ ID NO:20) |
| 24 | CG29 | YGEGT FTSDY SIYLD KKAQR DFIQW LLAQ GPSSGAPPPS (SEQ ID NO:21) |
| 26 | CG31 | YGEGT FTSDY SIYLD KKAQR LFIEW LLAQ GPSSGAPPPS (SEQ ID NO:22) |
| 27 | CG32 | YGQGT FTSDY SIYLD KKAQR DFVEW LLAQ GPSSGAPPPS (SEQ ID NO:23) |
| 28 | CG33 | YAEGT FTSDY SKLLE EEAVR LFIEW LLAG (SEQ ID NO:24) |
| 29 | CG34 | YGEGT FTSDY SIYLD KQAQR DFVEW LIAG GPSSGAPPPS (SEQ ID NO:25) |
| 30 | CG35 | YAEGT FTSDY SIYLD KLAQQ EFIDW LKAG GPSSGAPPPS (SEQ ID NO:26) |
| 32 | CG37 | YAEGT FTSDY SIYLD KLAQQ AFIEW LKAG GPSSGAPPPS (SEQ ID NO:27) |
| 33 | CG38 | YAEGT FTSDY SIYLD KLAQQ AFIEW LLAG GPSSGAPPPS (SEQ ID NO:28) |
| 34 | CG39 | YAEGT FTSDY SIYLD KIAQQ AFIEW LKAG GPSSGAPPPS (SEQ ID NO:29) |
| 35 | CG40 | HGEGT FTSDY SIYLD KIAQQ AFIEW LLAG GPSSGAPPPS (SEQ ID NO:30) |
| 36 | CG41 | HGQGT FTSDY SIYLD KIAQQ AFIEW LLAG GPSSGAPPPS (SEQ ID NO:31) |
| 38 | CG43 | YGEGT FTSDY SIYLD KKAQR DFVEW LLAG (SEQ ID NO:32) |
| 39 | CG44 | YGEGT FTSDY SIALD KKAQR DFIEW LLAG GPSSGAPPPS (SEQ ID NO:33) |
| 40 | CG45 | YGEGT FTSDY SIYLD KKAQR DFVEW LIAG (SEQ ID NO:34) |
| 41 | CG46 | YGEGT FTSDY SIYLD KKAQR DFIEW LIAG GPSSGAPPPS (SEQ ID NO:35) |
| 42 | CG47 | YGEGT FTSDY SIYLD KQAQR DFVEW LLAG GPSSGAPPPS (SEQ ID NO:36) |
| 43 | CG48 | YAEGT FTSDY SIYLD KIAQQ AFIEW LLAG GPSSGAPPPS (SEQ ID NO:37) |
| 47 | CG52 | YGEGT FTSDY SIYLD KQAQR DFVEW LLAG (SEQ ID NO:38) |
| 48 | CG53 | YGEGT FTSDY SIYLD KKAQQ DFVEW LIAG GPSSGAPPPS (SEQ ID NO:39) |
| 49 | CG54 | YGEGT FTSDY SIYLD KQAQRAFIEW LIAG GPSSGAPPPS (SEQ ID NO:40) |
| 50 | CG55 | YGEGT FTSDY SIYLD KLAQQ DFVEW LIAG GPSSGAPPPS (SEQ ID NO:41) |
| 51 | CG56 | YGEGT FTSDY SIALD KLAQQ DFIEW LLAG GPSSGAPPPS (SEQ ID NO:42) |
| 52 | CG57 | YGEGT FTSDY SIALD KKAQQ AFIEW LLAG GPSSGAPPPS (SEQ ID NO:43) |
| 54 | CG59 | YGEGT FTSDY SIYLD KLAQQ AFIEW LKAG GPSSGAPPPS (SEQ ID NO:44) |
| 55 | CG60 | YGEGT FTSDY SIYLD KLAQQ EFIDW LKAG GPSSGAPPPS (SEQ ID NO:45) |
| 56 | CG61 | YGEGT FTSDY SIYLD KIAQQ AFIEW LLAG GPSSGAPPPS (SEQ ID NO:46) |
| 58 | CG63 | YGEGT FTSDY SIYLD KLAQR DFVEW LLAG GPSSGAPPPS (SEQ ID NO:47) |
| 59 | CG64 | YGEGT FTSDY SIYLD KKAQQ DFVEW LLAG GPSSGAPPPS (SEQ ID NO:48) |
| 60 | CG65 | YGEGT FTSDY SIYLD KKAQQ AFVEW LLAG GPSSGAPPPS (SEQ ID NO:49) |
| 61 | CG66 | YGEGT FTSDY SIYLD KLAQQ AFVEW LLAG GPSSGAPPPS (SEQ ID NO:50) |
| 62 | CG67 | YSEGT FTSDY SIYLD KKAQR DFVEW LLAG GPSSGAPPPS (SEQ ID NO:51) |
| 63 | CG68 | YGEGT FTSDY SIYLD KKAQR DFVEW LVAG GPSSGAPPPS (SEQ ID NO:52) |
| 64 | CG69 | YGEGT FTSDY SIYLD KLAQQ DFVEW LLAG GPSSGAPPPS (SEQ ID NO:53) |
| 65 | CG70 | YGEGT FTSDY SIYLD KKAQR EFVEW LLAG GPSSGAPPPS (SEQ ID NO:54) |
| 66 | CG71 | YGEGT FTSDY SIYLD KKAQR EFIDW LLAG GPSSGAPPPS (SEQ ID NO:55) |
| 67 | CG72 | YGEGT FTSDY SIYLD KKAQR DFIDW LLAG GPSSGAPPPS (SEQ ID NO:56) |
| 68 | CG73 | YGEGT FTSDY SIYLD KKAQR AFIDW LLAG GPSSGAPPPS (SEQ ID NO:57) |
| 69 | CG74 | YGEGT FTSDY SIYLD KKAQR DFIEW LIAQ GPSSGAPPPS (SEQ ID NO:58) |
| 70 | CG75 | YGEGT FTSDY SIYLD KKAQR DFVEW LIAQ GPSSGAPPPS (SEQ ID NO:59) |
| 71 | CG76 | YGEGT FTSDY SIYLD KKAQR DFVQW LLAG GPSSGAPPPS (SEQ ID NO:60) |
| 72 | CG77 | YGEGT FTSDY SIILD KKAQR DFVEW LLAG GPSSGAPPPS (SEQ ID NO:61) |
| 73 | CG78 | YGEGT FTSDY SIALD KKAQR DFIEW LIAG GPSSGAPPPS (SEQ ID NO:62) |
| 74 | CG79 | YGEGT FTSDY SIYLD KKAQR DFIEW LLAG (SEQ ID NO:63) |
| 75 | CG80 | YGEGT FTSDY SIYLD KKAQR DFIEY LLAG (SEQ ID NO:64) |
| 76 | CG81 | YGEGT FTSDY SIALD KQAQR DFVEW LIAG GPSSGAPPPS (SEQ ID NO:65) |
| 77 | CG82 | YGEGT FTSDY SIYLD KQAQR DFVEW LLAQ GPSSGAPPPS (SEQ ID NO:66) |
| 78 | CG83 | YGEGT FTSDY SIYLD KIAQR DFVEW LIAG GPSSGAPPPS (SEQ ID NO:67) |
| 79 | CG84 | YGEGT FTSDY SIYLD KQHQR DFVEW LIAG GPSSGAPPPS (SEQ ID NO:68) |
| 80 | CG85 | YGEGT FTSDY SIYLD KKAQR DFINW LIAG GPSSGAPPPS (SEQ ID NO:69) |
| 81 | CG86 | YGEGT FTSDY SIYLD KKHQR DFIEW LIAG GPSSGAPPPS (SEQ ID NO:70) |
| 82 | CG87 | YGEGT FTSDY SIYLD KIAQR DFIEW LIAG GPSSGAPPPS (SEQ ID NO:71) |
| 83 | CG88 | YGEGT FTSDY SIYLD KKAQQ DFIEW LIAG GPSSGAPPPS (SEQ ID NO:72) |
| 84 | CG89 | YGEGT FTSDY SIALD KQAQR DFVEW LLAG GPSSGAPPPS (SEQ ID NO:73) |
| 85 | CG90 | YGEGT FTSDY SIYLD KQAQQ DFVEW LLAG GPSSGAPPPS (SEQ ID NO:74) |
| 86 | CG91 | YGEGT FTSDY SIYLD KIAQR DFVEW LLAG GPSSGAPPPS (SEQ ID NO:75) |
| 87 | CG92 | YGEGT FTSDY SIYLD KQAQR DFVNW LLAG GPSSGAPPPS (SEQ ID NO:76) |
| 88 | CG93 | YGEGT FTSDY SIYLD KIAQQ AFVEW LLAG GPSSGAPPPS (SEQ ID NO:77) |
| 89 | CG94 | YGEGT FTSDY SIYMD KLAQQ AFVEW LLAG GPSSGAPPPS (SEQ ID NO:78) |
| 90 | CG95 | YGEGT FTSDY SIYLD KLAQQ AFVNW LLAG GPSSGAPPPS (SEQ ID NO:79) |
| 91 | CG96 | YSEGT FTSDY SIYLD KIAQR DFVEW LLAG GPSSGAPPPS (SEQ ID NO:80) |
| 92 | CG97 | YSEGT FTSDY SIALD KKAQR DFVEW LLAG GPSSGAPPPS (SEQ ID NO:81) |
| 93 | CG98 | YSEGT FTSDY SIYLD KKAQQ DFVEW LLAG GPSSGAPPPS (SEQ ID NO:82) |
| 94 | CG99 | YGEGT FTSDY SIALD KKAQR DFIDW LLAG GPSSGAPPPS (SEQ ID NO:83) |
| 95 | CG100 | YGEGT FTSDY SIYLD KKAQR DFINW LLAG GPSSGAPPPS (SEQ ID NO:84) |
| 96 | CG101 | YGEGT FTSDY SIYLD KKAQQ DFIDW LLAG GPSSGAPPPS (SEQ ID NO:85) |
| 97 | CG102 | YGEGT FTSDY SIYLD KKAQR DFINW LIAQ GPSSGAPPPS (SEQ ID NO:86) |
| 98 | CG103 | YGEGT FTSDY SIALD KKAQR DFIEW LIAQ GPSSGAPPPS (SEQ ID NO:87) |
| 99 | CG104 | YGEGT FTSDY SIYLD KKAQQ DFIEW LIAQ GPSSGAPPPS (SEQ ID NO:88) |
| 100 | CG105 | YGEGT FTSDY SIYLD KIAQR DFIEW LIAQ GPSSGAPPPS (SEQ ID NO:89) |
| 101 | CG106 | YGEGT FTSDY SILLD KKAQR DFVEW LLAG GPSSGAPPPS (SEQ ID NO:90) |
| 102 | CG107 | YGEGT FTSDY SIYLD KKAQH DFVEW LLAG GPSSGAPPPS (SEQ ID NO:91) |
| 107 | CG112 | YGEGT FTSDY SIYLD KQAQH AFIEW LIAG GPSSGAPPPS (SEQ ID NO:92) |
| 108 | CG113 | HSEGT FTSDY SILLD KIAQQ DFVEW LLAG GPSSGAPPPS (SEQ ID NO:93) |
| 109 | CG114 | HSEGT FTSDY SILLD KIAQH DFIEW LIAG GPSSGAPPPS (SEQ ID NO:94) |
| 110 | CG115 | YSEGT FTSDY SILLD KIAQR EFIEW LLAG GPSSGAPPPS (SEQ ID NO:95) |
| 111 | CG116 | HSEGT FTSDY SILLD KIAQR EFIEW LLAG GPSSGAPPPS (SEQ ID NO:96) |
| 112 | CG117 | HSEGT FTSDY SILLD KIAQR EFIEW LLEG GPSSGAPPPS (SEQ ID NO:97) |
| 113 | CG118 | HSEGT FTSDY SILLD KIAQK EFIEW LLAG GPSSGAPPPS (SEQ ID NO:98) |
| 114 | CG119 | YSEGT FTSDY SILLD KIAQK EFIEW LLAG GPSSGAPPPS (SEQ ID NO:99) |
| 115 | CG120 | HSEGT FTSDY SILLD KIAQK EFIEW LLEG GPSSGAPPPS (SEQ ID NO:100) |
| 116 | CG121 | YSEGT FTSDY SILLD KIAQRAFIEW LLAG GPSSGAPPPS (SEQ ID NO:101) |
| 117 | CG122 | YSEGT FTSDY SILLD KIAQR EFIEW LIAG GPSSGAPPPS (SEQ ID NO:102) |
| 118 | CG123 | YSEGT FTSDY SILLD KIAQR EFVQW LLAG GPSSGAPPPS (SEQ ID NO:103) |
| 119 | CG124 | YSEGT FTSDY SILLD KIAQR EFVQW LIAG GPSSGAPPPS (SEQ ID NO:104) |
| 120 | CG125 | YSEGT FTSDY SILLD KIAQRAFVQW LIAG GPSSGAPPPS (SEQ ID NO:105) |
| 121 | CG126 | YGEGT FTSDY SILLD KIAQR EFIEW LLAG GPSSGAPPPS (SEQ ID NO:106) |
| 122 | CG127 | YSEGT FTSDY SILLD KKAQR DFIEW LLAG GPSSGAPPPS (SEQ ID NO:107) |
| 123 | CG128 | YGEGT FTSDY SILLD KKAQR DFIEW LLAG GPSSGAPPPS (SEQ ID NO:108) |
| 124 | CG129 | YSEGT FTSDY SILLD KKAQR DFIEW LIAG GPSSGAPPPS (SEQ ID NO:109) |
| 125 | CG130 | YSEGT FTSDY SILLD KKAQR DFVQW LIAG GPSSGAPPPS (SEQ ID NO:110) |
| 126 | CG131 | YSEGT FTSDY SILLD KIAQR DFVEW LIAG GPSSGAPPPS (SEQ ID NO:111) |
| 127 | CG132 | YGEGT FTSDY SILLD KIAQR DFVEW LIAG GPSSGAPPPS (SEQ ID NO:112) |
| 128 | CG133 | YSEGT FTSDY SILLD KIAQQ DFVEW LLAG GPSSGAPPPS (SEQ ID NO:117) |
| 129 | CG134 | YSEGT FTSDY SILLD KIAQQ EFIEW LLAG GPSSGAPPPS (SEQ ID NO:118) |
| 130 | CG135 | YSEGT FTSDY SILLD KIAQQ EFIEW LIAG GPSSGAPPPS (SEQ ID NO:119) |
| 131 | CG136 | YSEGT FTSDY SILLD KKAQQ DFIEW LLAG GPSSGAPPPS (SEQ ID NO:120) |
| 132 | CG137 | YSEGT FTSDY SILLD KKAQQ DFIEW LIAG GPSSGAPPPS (SEQ ID NO:121) |

According to a specific embodiment of the present invention, the GLP-1/GIP dual-target polypeptide has the amino acid sequence shown below: SEQ ID NO:2, SEQ ID NO: 13, SEQ ID NO: 16, SEQ ID NO:25-28, SEQ ID NO:37, SEQ ID NO:50-51, SEQ ID NO:56, SEQ ID NO:58, SEQ ID NO:83, SEQ ID NO:85, SEQ ID NO:93, SEQ ID NO:95, SEQ ID NO: 101-107, SEQ ID NO:110-112.

The present invention provides a fusion protein, comprising: (1) a GLP-1/GIP dual-target polypeptide described above; (2) an Fc fragment, the C-terminus of the GLP-1/GIP dual-target polypeptide is connected with the N-terminus of the Fc fragment. The fusion protein according to the embodiments of the present invention also has excellent binding activity to GLP-1 and/or GIP receptors, has dual functions of weight loss and hypoglycemia, and can effectively control or reduce weight and blood glucose levels.

According to a specific embodiment of the present invention, the Fc fragment is an Fc fragment of human IgG4 or a mutant thereof. Fusion of the Fc fragment of immunoglobulin on the GLP-1/GIP dual-target polypeptide can extend the half-life of the fusion protein in vivo.

According to a specific embodiment of the present invention, the Fc fragment comprises an amino acid sequence shown in SEQ ID NO:113.

GluSerLysTyrGlyProProCysProProCysProAlaProGluAlaAlaGlyGlyProSerValPheL euPheProProLysProLysAspThrLeuMetIle SerArgThrProGluValThrCysVal Val ValAspVal SerGlnGl uAspProGluValGlnPheAsnTrpTyrValAspGlyValGluValHisAsnAlaLysThrLysProArgGluGluGlnP heAsnSerThrTyrArgValValSerValLeuThrValLeuHisGlnAspTrpLeuAsnGlyLysGluTyrLysCysLys ValSerAsnLysGlyLeuProSerSerIleGluLysThrIleSerLysAlaLysGlyGlnProArgGluProGlnValTyrTh rLeuProProSerGlnGluGluMetThrLysAsnGlnValSerLeuThrCysLeuValLysGlyPheTyrProSerAspIl eAlaValGluTrpGluSerAsnGlyGlnProGluAsnAsnTyrLysThrThrProProValLeuAspSerAspGlySerP hePheLeuTyrSerArgLeuThrValAspLysSerArgTrpGlnGluGlyAsnValPheSerCysSerValMetHisGlu AlaLeuHisAsnHisTyrThrGlnLysSerLeuSerLeuSerLeuGly (SEQ ID NO: 113).

According to a specific embodiment of the present invention, the fusion protein further comprises a linker peptide, and the linker peptide has the amino acid sequence shown in SEQ ID NO: 114.

GlyGlyGlyGlySerGlyGlyGlyGlySerGlyGlyGlyGlySerAla (SEQ ID NO: 114).

According to a specific embodiment of the present invention, the N-terminus of the linker peptide is connected with the C-terminal of the GLP-1/GIP dual-target polypeptide, and the C-terminal of the linker peptide is connected with the N-terminal of the Fc fragment.

The present invention provides a nucleic acid molecule encoding the GLP-1/GIP dual-target polypeptide or fusion protein described above. The GLP-1/GIP dual-target polypeptide or fusion protein encoded by the nucleic acid molecule according to the embodiments of the present invention also has excellent binding activity with GLP-1 and/or GIP receptors, and has the dual functions of weight loss and hypoglycemia, and can effectively control or reduce weight and blood glucose levels.

The present invention provides an expression vector. According to the embodiments of the present invention, the expression vector comprises the aforementioned nucleic acid molecule encoding the GLP-1/GIP dual-target polypeptide or fusion protein. When connecting the above-mentioned nucleic acid molecule to the vector, the nucleic acid molecule can be directly or indirectly connected with the control elements on the vector, as long as these control elements can control the translation and expression of the nucleic acid molecule. Of course, these control elements can come directly from the vector itself, or they can be exogenous, that is, they do not come from the vector itself. Of course, it suffices that the nucleic acid molecule is operably connected with the control element. In this article, "operably connected" refers to connecting an exogenous gene to a vector so that the control elements in the vector, such as transcription control sequences and translation control sequences, can exert their expected functions of regulating the transcription and translation of the exogenous gene.

According to a specific embodiment of the present invention, the expression vector is a eukaryotic expression vector.

The present invention provides a recombinant cell. According to the embodiments of the present invention, the recombinant cells carry the nucleic acid molecule encoding the GLP-1/GIP dual-target polypeptide or the fusion protein described above, or the expression vector described above. The recombinant cells according to specific embodiments of the present invention can express the GLP-1/GIP dual-target polypeptide or fusion protein, and the GLP-1/GIP dual-target polypeptide and fusion protein have excellent binding activity to GLP-1 and/or GIP receptors, and has dual functions of weight loss and hypoglycemia, and can effectively control or reduce body weight and blood glucose levels.

According to a specific embodiment of the present invention, the recombinant cells are mammalian cells, such as CHO cells.

According to a specific embodiment of the present invention, the recombinant cells do not comprise animal germ cells, fertilized eggs or embryonic stem cells.

The present invention provides a pharmaceutical composition, comprising the aforementioned GLP-1/GIP dual-target polypeptide, the aforementioned fusion protein, the aforementioned nucleic acid molecule encoding the aforementioned GLP-1/GIP dual-target polypeptide or fusion protein, the aforementioned expression vector, or the aforementioned recombinant cell. The pharmaceutical composition may comprise a pharmaceutically acceptable adjuvant, and the pharmaceutically acceptable adjuvant comprises at least one of stabilizers, wetting agents, emulsifiers, binders, isotonic agents; the pharmaceutical composition is at least one kind of tablets, granules, powders, capsules, solutions, suspensions or lyophilized preparations. The pharmaceutical compositions according to specific embodiments of the present invention are used to treat diabetes, obesity, fatty liver disease, non-alcoholic fatty liver disease, non-alcoholic steatohepatitis, dyslipidemia, metabolic syndrome and other diseases, and have long-term weight loss and/or hypoglycemic functions, and can effectively control or reduce body weight and blood glucose levels.

The present invention provides use of the aforementioned GLP-1/GIP dual-target polypeptide, the aforementioned fusion protein, the aforementioned nucleic acid molecule encoding the aforementioned GLP-1/GIP dual-target polypeptide or fusion protein, the aforementioned expression vector, and the aforementioned recombinant cells in the manufacture of a medicament. According to specific embodiments of the present invention, the medicament is used to control or reduce blood glucose and weight.

The present invention provides a method for preparing the aforementioned fusion protein, the method comprises: 1) constructing the aforementioned expression vector; 2) introducing the expression vector into host cells to obtain recombinant cells to express the aforementioned fusion protein. The fusion protein prepared according to the method of specific embodiments of the present invention has excellent binding activity to GLP-1 and/or GIP receptors, has dual functions of weight loss and hypoglycemia, and can effectively control or reduce body weight and blood glucose levels.

Furthermore, the "GLP-1/GIP dual-target polypeptide" or "recombinant polypeptide" described in the present invention can not only be prepared by recombinant expression, but also can be prepared by chemical synthesis. No matter what preparation method is used, as long as it has one of the hypoglycemic or weight-loss activities described in the present invention, it is within the protection scope of the present invention.

According to a specific embodiment of the present invention, the recombinant cells do not comprise animal germ cells, fertilized eggs or embryonic stem cells.

The present invention provides a method for reducing blood glucose and/or body weight of a patient, comprising administering to the patient at least one of the following: 1) the aforementioned GLP-1/GIP dual-target polypeptide; 2) the aforementioned fusion protein; 3) the aforementioned nucleic acid molecule encoding the GLP-1/GIP dual-target polypeptide or fusion protein; 4) the aforementioned expression vector; 5) the aforementioned recombinant cell; and 6) the aforementioned pharmaceutical composition. The methods according to specific embodiments of the present invention can effectively and long-term control or reduce the weight and/or blood glucose levels of patients, treat fatty liver disease, non-alcoholic fatty liver disease, non-alcoholic steatohepatitis, dyslipidemia and metabolic syndrome and other diseases. The "Fc fragment" of the present invention can be a human IgG4 Fc fragment, or a variant of the IgG4 Fc fragment. The mutant has one or more amino acid site mutations compared to the wild-type IgG4 Fc.

The names of the GLP-1/GIP dual-target polypeptides of the present invention begin with "CG", such as "CG01", "CG02", etc., and the names of the GLP-1/GIP dual-target fusion proteins begin with "HEC-G", such as " HEC-G01", "HEC-G02", etc.

The present invention will be described below with reference to specific examples. It should be noted that the experimental methods used in the following examples are all conventional methods unless otherwise specified. The materials, reagents, etc. used in the following examples can all be obtained from commercial sources unless otherwise specified. These examples are only descriptive and do not limit the present invention in any way.

### Example 1

This example provides a method for synthesizing the polypeptide of the present invention.

1. Solid-phase peptide synthesis was performed on a peptide synthesizer: resin was added into a 150ml reactor, then 50ml of dichloromethane (DCM) was added and the resin was soaked for 2 h. The resin was washed with *N, N*-dimethylformamide (DMF) and then drained, and this was repeated four times, then the resin was drained. The first amino acid at the C-terminus of Fmoc (protected), DCM and *N,N*-diisopropylethylamine (DIEA) were weighed and added to the reactor, then the reactor was placed in a shaker at 30°C for 2 h. The mixture was blocked with methanol solution (methanol: DIEA: DCM=1:1:2) for 0.5 h, then washed with DMF four times and then drained. 20% piperidine solution was added the reactor to remove the Fmoc protecting group. After deprotection, the mixture was washed with DMF four times and then drained.

2. The second amino acid at the C-terminus of Fmoc (protected), 1-hydroxybenzotriazole (HOBT) and *N, N'*-diisopropylcarbodiimide (DIC) were weighed and added to the reactor, then the reactor was placed in a shaker at 30°C for 1 h. A small amount of resin was taken and tested with the ninhydrin method. If the resin has color, it means the condensation is incomplete and the reaction continues. After the reaction was complete, the resin was washed with DMF four times and then drained. A certain amount of 20% piperidine (piperidine/DMF=1:4) was added to the reactor, then the reactor was placed on a decolorizing shaker and shaken for 20 min to remove the Fmoc protecting group on the resin. After deprotection, the resin was washed with DMF four times, then drained to check whether the protecting group was removed.

3. The amino acids were connected in sequence according to step 2, and finally a cleavage reagent was used to remove all the polypeptide protective groups, then the polypeptide was cut from the resin, and send for purification.

4. After the target peptide was synthesized, the target peptide was separated from impurities through reversed-phase liquid chromatography purification. The collected target peptide was freeze-dried into powder and sent to QC for quality inspection and purity and mass spectrometry identification. After HPLC testing, the purity was greater than 95%. The molecular weight of the peptides identified by mass spectrometry was consistent with the theoretical molecular weight. Table 2 shows the synthesized polypeptide compounds.

**Table 2:**

| Name of the polypeptide | Sequences of GLP-1/GIP dual-target polypeptide |
|---|---|
| CG113 | HSEGT FTSDY SILLD KIAQQ DFVEW LLAG GPSSGAPPPS (SEQ ID NO:93) |
| CG115 | YSEGT FTSDY SILLD KIAQR EFIEW LLAG GPSSGAPPPS (SEQ ID NO:95) |
| CG121 | YSEGT FTSDY SILLD KIAQRAFIEW LLAG GPSSGAPPPS (SEQ ID NO:101) |
| CG122 | YSEGT FTSDY SILLD KIAQR EFIEW LIAG GPSSGAPPPS (SEQ ID NO:102) |
| CG123 | YSEGT FTSDY SILLD KIAQR EFVQW LLAG GPSSGAPPPS (SEQ ID NO:103) |
| CG124 | YSEGT FTSDY SILLD KIAQR EFVQW LIAG GPSSGAPPPS (SEQ ID NO:104) |
| CG125 | YSEGT FTSDY SILLD KIAQR AFVQW LIAG GPSSGAPPPS (SEQ ID NO:105) |
| CG126 | YGEGT FTSDY SILLD KIAQR EFIEW LLAG GPSSGAPPPS (SEQ ID NO:106) |
| CG127 | YSEGT FTSDY SILLD KKAQR DFIEW LLAG GPSSGAPPPS (SEQ ID NO:107) |
| CG133 | YSEGT FTSDY SILLD KIAQQ DFVEW LLAG GPSSGAPPPS (SEQ ID NO:117) |
| CG134 | YSEGT FTSDY SILLD KIAQQ EFIEW LLAG GPSSGAPPPS (SEQ ID NO:118) |
| CG135 | YSEGT FTSDY SILLD KIAQQ EFIEW LIAG GPSSGAPPPS (SEQ ID NO:119) |
| CG136 | YSEGT FTSDY SILLD KKAQQ DFIEW LLAG GPSSGAPPPS (SEQ ID NO:120) |
| CG137 | YSEGT FTSDY SILLD KKAQQ DFIEW LIAG GPSSGAPPPS (SEQ ID NO:121) |

### Example 2 Determination of in vitro activity of polypeptides

HEK293 cells expressing GLP-1R or GIPR were treated with peptide samples, human GLP-1 (purchased) and human GIP (purchased), respectively, the specific operations are as follows:
1) The genes GIPR and GLP-1R were optimized and routinely synthesized at Genewiz, and the genes were cloned into the vector pUC57-Amp to prepare mini-scale recombinant plasmid DNA and puncture bacteria containing the recombinant plasmid;
2) pUC57-GIPR recombinant plasmid DNA was double-digested with *Hind*III and *EcoR* I, and pUC57-GLP-1R was double-digested with H*in*dIII and X*ho*I*.* The digested product was electrophoresed on 1% agarose gel and the target band was cut out with a clean blade, then a gel recovery kit was used to recover the target fragment. The specific experimental operations are carried out according to the kit instructions;
3) The target fragment enzymatic digestion recovery product and vector plasmid pcDNA3.1 fragment were connected through T4 ligase and transformed into DH5α competent cells, then single colonies were isolated by spreading on plates, and the transformants were selected and expanded for enzyme digestion verification and sequencing verification.
4) 200mL of the bacterial liquid obtained in inoculation step 3) and verified by sequencing to contain the fusion protein of the target product was used for plasmid extraction. The kit used was PureLink HiPure Plasmid Maxiprep Kit, and according to the instructions. After the plasmid was verified to be correct by PCR and enzyme digestion, pvul restriction enzyme was used for linearization. Finally, the ethanol precipitation method was used to recover the plasmid.
5) The host cells were HEK293, one day before transfection, the cells were spread into a 6-well plate at a density of 2x10^6 cells/well, and the addition volume was 1mL/well. The recovered linearized plasmid was transfected into HEK293 cells using Lipofectamine 3000 transfection method, then G418 was added to screen to obtain mixed strains, single clones were obtained by limiting dilution and isolation, and the activity was tested and verified.

Using a cAMP detection kit (Cisbio, 62AM6PEC) to detect cAMP produced by the recipient cells according to the steps described in the operating instructions. The specific steps are as follows:
1) Preparation of Assay buffer: To the complete culture medium (DMEM medium + 10% FBS) was added 4/1000 of 500 mM IBMX stock solution, cAMP-d2 working solution and anti-cAMP-crytate working solution were prepared according to the kit instructions;
2) The test sample and control sample human GLP-1 (purchased) and GIP (purchased) were diluted to a stock solution with an initial concentration of 500 nM, and then 20 µL of which was added to 80 µL Assay buffer (diluted 5 times) in a gradient step-by-step dilution, a total of 8 compound gradients including stock solution were obtained;
3) Preparation of cell suspension: The cells HEK293-GLP-1R and HEK293-GIPR were taken out from a liquid nitrogen tank, and placed in a 37°C water bath immediately. If the mixture was not completely melted within 1.5 min, the cells were added dropwise to a 15mL centrifuge tube containing 8mL warm culture medium on a clean bench, then centrifuged at 900rpm for 5 min, the supernatant was discarded, and the cells were re-suspended with 1mL complete culture medium (15 times by pipetting). 20µL of the suspension was taken immediately and mixed with an equal volume of trypan blue, the cells were diluted to 4x10^5 cells/mL after taking 20 µL to count the number of viable cells.
4) A 384-well plate was divided into GLP-1R cell area and GIPR cell area, cell suspension was added to wells in the corresponding area using a 12-channel adjustable dispenser with 5 µL per well, and then the serial dilutions of the test substance and positive control substance were added into the 384-well plate corresponding to the cells using a 12-channel adjustable dispenser with 5 µL per well (samples of the same concentration were replicated in 2 parallel wells); negative control: 10 µL assay buffer /well, each 384-well plate was set 3 wells and covered with a white sealing film, and placed in a 37°C constant temperature incubator, then taken out after 0.5 h;
5) The cAMP-d2 working solution and anti-cAMP-crytate working solution were diluted 20-fold with lysis buffer in the Hi-range kit before use, and then mixed evenly according to 1: 1 to prepare the cAMP detection reagent mixture, cAMP detection reagent mixture was added with 10 µL/well of the sample group, and 5 µL of lysis buffer and 5 µL of diluted anti-cAMP-crytate working solution were added to each well of the negative control, then the plate was covered with a white lid, and placed at room temperature in the dark for 1 h.
6) The fluorescence values at 665nm and 620nm were detected by a multifunctional microplate reader;
7) Using this to establish a dose-response curve, then EC50 value was calculated, and compared each other.

The specific results are shown in Table 3. All peptides showed strong GIPR agonistic activity, but there were certain differences in GLP-1R agonistic activity. Among them, CG133 and CG134 had weaker GLP-1R agonistic activities.

**Table 3:**

| Name of the GLP-1/GIP peptide | Ec50(nM) of the peptide | |
|---|---|---|
| | GLP-1R | GIPR |
| CG113 | 0.164 | 0.067 |
| CG115 | 0.291 | 0.005 |
| CG121 | 0.322 | 0.018 |
| CG122 | 0.191 | 0.016 |
| CG123 | 0.879 | 0.044 |
| CG124 | 0.232 | 0.041 |
| CG125 | 0.205 | 0.073 |
| CG126 | 0.918 | 0.027 |
| CG127 | 0.364 | 0.084 |
| CG133 | 8.181 | 0.017 |
| CG134 | 2.146 | 0.012 |
| CG135 | 0.558 | 0.015 |
| CG136 | 1.107 | 0.050 |
| CG137 | 0.250 | 0.061 |
| Human GLP-1 | 0.082 | N/A |
| Human GIP | N/A | 0.119 |

### Example 3 Construction of expression vector

This example used a molecular cloning method to fuse the GLP-1/GIP dual receptor agonist polypeptide with the Fc fragment with a linking peptide. IIWherein, the amino acid sequence of the wild-type GLP-1 was: HAEGT FTSDV SSYLE GQAAK EFIAW LVKGR G (SEQ ID NO: 115), the amino acid sequence of the wild-type GIP was: YAEGT FISDY SIAMD KIHQQ DFVNW LLAQ (SEQ ID NO: 116), and the GLP-1/GIP dual receptor agonist polypeptide was obtained by mutating the amino acid sequence of wild-type GLP-1 and GIP; the synthesized sequence was double digested and inserted into the same enzyme digestion site of the mammalian cell expression vector; or site-directed mutation primers were designed based on the existing vectors, and a series of mutant vectors were constructed through polymerase chain reaction (PCR), then sent to a sequencing company and verified the sequencings were correct, and an endotoxin-removing plasmid extraction kit (Endo-free Plasmid Mini kit) from OMEGA was used to extract the plasmid vector, the Cat. No. was D6950-01, and the plasmid vector was stored at - 20°C for later use. Table 4 shows the GLP-1/GIP dual-target polypeptide sequence in the GLP-1/GIP dual-target fusion protein.

**Table 4:**

| No. | Name of the fusion protein | Amino acid sequences of dual-target polypeptide in fusion protein |
|---|---|---|
| 1 | HEC-G01 | YAEGT FTSDY SIYLD KQAAK EFVNW LLAG GPSSGAPPPS (SEQ ID NO:2) |
| 2 | HEC-G02 | YGEGT FTSDY SIYLD KQAAK EFVNW LLAG GPSSGAPPPS (SEQ ID NO:3) |
| 3 | HEC-G03 | YGEGT FTSDY SIALD KQAAK EFVNW LLAG GPSSGAPPPS (SEQ ID NO:4) |
| 6 | HEC-G06 | YGEGT FTSDY SIYLD KKAQR DFVEW LLAQ GPSSGAPPPS (SEQ ID NO:5) |
| 7 | HEC-G07 | YGEGT FTSDY SIALD KIAQK AFVQW LIAG GPSSGAPPPS (SEQ ID NO:6) |
| 8 | HEC-G08 | YGEGT FTSDY SIYLD KIAQKAFVQW LIAG GPSSGAPPPS (SEQ ID NO:7) |
| 9 | HEC-G09 | YGEGT FTSDY SIALD KIAAK AFVQW LIAG GPSSGAPPPS (SEQ ID NO:8) |
| 10 | HEC-G10 | YGEGT FTSDY SIALD KQAAKAFVQW LIAG GPSSGAPPPS (SEQ ID NO:9) |
| 11 | HEC-G11 | YGEGT FTSDY SIALD KQRAK DFVQW LIAG GPSSGAPPPS (SEQ ID NO:10) |
| 13 | HEC-G13 | YSEGT FTSDY SKLLE EEAVR LFIEW LLAG (SEQ ID NO:11) |
| 14 | HEC-G14 | YSEGT FTSDY SKLLE EEAVR LFIEW LVKG (SEQ ID NO:12) |
| 15 | HEC-G20 | YGEGT FTSDY SIYLD KKAQR DFVEW LLAG GPSSGAPPPS (SEQ ID NO:13) |
| 16 | HEC-G21 | YGEGT FTSDY SIYLD KKAQR DFVEW LKAG GPSSGAPPPS (SEQ ID NO:14) |
| 17 | HEC-G22 | YGEGT FTSDY SIYLD KKAQR DFVEW LVKG GPSSGAPPPS (SEQ ID NO:15) |
| 18 | HEC-G23 | YGEGT FTSDY SIYLD KKAQR DFVEW LIAG GPSSGAPPPS (SEQ ID NO:16) |
| 19 | HEC-G24 | YGEGT FTSDY SIALD KKAQR DFVEW LLAQ GPSSGAPPPS (SEQ ID NO:17) |
| 20 | HEC-G25 | YGEGT FTSDY SIALD KKAQR DFIEW LLAQ GPSSGAPPPS (SEQ ID NO:18) |
| 21 | HEC-G26 | YGEGT FISDY SIYLD KKAQR DFVEW LLAQ GPSSGAPPPS (SEQ ID NO:19) |
| 23 | HEC-G28 | YGEGT FTSDY SIYLD KKAQR DFVQW LLAQ GPSSGAPPPS (SEQ ID NO:20) |
| 24 | HEC-G29 | YGEGT FTSDY SIYLD KKAQR DFIQW LLAQ GPSSGAPPPS (SEQ ID NO:21) |
| 26 | HEC-G31 | YGEGT FTSDY SIYLD KKAQR LFIEW LLAQ GPSSGAPPPS (SEQ ID NO:22) |
| 27 | HEC-G32 | YGQGT FTSDY SIYLD KKAQR DFVEW LLAQ GPSSGAPPPS (SEQ ID NO:23) |
| 28 | HEC-G33 | YAEGT FTSDY SKLLE EEAVR LFIEW LLAG (SEQ ID NO:24) |
| 29 | HEC-G34 | YGEGT FTSDY SIYLD KQAQR DFVEW LIAG GPSSGAPPPS (SEQ ID NO:25) |
| 30 | HEC-G35 | YAEGT FTSDY SIYLD KLAQQ EFIDW LKAG GPSSGAPPPS (SEQ ID NO:26) |
| 32 | HEC-G37 | YAEGT FTSDY SIYLD KLAQQ AFIEW LKAG GPSSGAPPPS (SEQ ID NO:27) |
| 33 | HEC-G38 | YAEGT FTSDY SIYLD KLAQQ AFIEW LLAG GPSSGAPPPS (SEQ ID NO:28) |
| 34 | HEC-G39 | YAEGT FTSDY SIYLD KIAQQ AFIEW LKAG GPSSGAPPPS (SEQ ID NO:29) |
| 35 | HEC-G40 | HGEGT FTSDY SIYLD KIAQQ AFIEW LLAG GPSSGAPPPS (SEQ ID NO:30) |
| 36 | HEC-G41 | HGQGT FTSDY SIYLD KIAQQ AFIEW LLAG GPSSGAPPPS (SEQ ID NO:31) |
| 38 | HEC-G43 | YGEGT FTSDY SIYLD KKAQR DFVEW LLAG (SEQ ID NO:32) |
| 39 | HEC-G44 | YGEGT FTSDY SIALD KKAQR DFIEW LLAG GPSSGAPPPS (SEQ ID NO:33) |
| 40 | HEC-G45 | YGEGT FTSDY SIYLD KKAQR DFVEW LIAG (SEQ ID NO:34) |
| 41 | HEC-G46 | YGEGT FTSDY SIYLD KKAQR DFIEW LIAG GPSSGAPPPS (SEQ ID NO:35) |
| 42 | HEC-G47 | YGEGT FTSDY SIYLD KQAQR DFVEW LLAG GPSSGAPPPS (SEQ ID NO:36) |
| 43 | HEC-G48 | YAEGT FTSDY SIYLD KIAQQ AFIEW LLAG GPSSGAPPPS (SEQ ID NO:37) |
| 47 | HEC-G52 | YGEGT FTSDY SIYLD KQAQR DFVEW LLAG (SEQ ID NO:38) |
| 48 | HEC-G53 | YGEGT FTSDY SIYLD KKAQQ DFVEW LIAG GPSSGAPPPS (SEQ ID NO:39) |
| 49 | HEC-G54 | YGEGT FTSDY SIYLD KQAQRAFIEW LIAG GPSSGAPPPS (SEQ ID NO:40) |
| 50 | HEC-G55 | YGEGT FTSDY SIYLD KLAQQ DFVEW LIAG GPSSGAPPPS (SEQ ID NO:41) |
| 51 | HEC-G56 | YGEGT FTSDY SIALD KLAQQ DFIEW LLAG GPSSGAPPPS (SEQ ID NO:42) |
| 52 | HEC-G57 | YGEGT FTSDY SIALD KKAQQ AFIEW LLAG GPSSGAPPPS (SEQ ID NO:43) |
| 54 | HEC-G59 | YGEGT FTSDY SIYLD KLAQQ AFIEW LKAG GPSSGAPPPS (SEQ ID NO:44) |
| 55 | HEC-G60 | YGEGT FTSDY SIYLD KLAQQ EFIDW LKAG GPSSGAPPPS (SEQ ID NO:45) |
| 56 | HEC-G61 | YGEGT FTSDY SIYLD KIAQQ AFIEW LLAG GPSSGAPPPS (SEQ ID NO:46) |
| 58 | HEC-G63 | YGEGT FTSDY SIYLD KLAQR DFVEW LLAG GPSSGAPPPS (SEQ ID NO:47) |
| 59 | HEC-G64 | YGEGT FTSDY SIYLD KKAQQ DFVEW LLAG GPSSGAPPPS (SEQ ID NO:48) |
| 60 | HEC-G65 | YGEGT FTSDY SIYLD KKAQQ AFVEW LLAG GPSSGAPPPS (SEQ ID NO:49) |
| 61 | HEC-G66 | YGEGT FTSDY SIYLD KLAQQ AFVEW LLAG GPSSGAPPPS (SEQ ID NO:50) |
| 62 | HEC-G67 | YSEGT FTSDY SIYLD KKAQR DFVEW LLAG GPSSGAPPPS (SEQ ID NO:51) |
| 63 | HEC-G68 | YGEGT FTSDY SIYLD KKAQR DFVEW LVAG GPSSGAPPPS (SEQ ID NO:52) |
| 64 | HEC-G69 | YGEGT FTSDY SIYLD KLAQQ DFVEW LLAG GPSSGAPPPS (SEQ ID NO:53) |
| 65 | HEC-G70 | YGEGT FTSDY SIYLD KKAQR EFVEW LLAG GPSSGAPPPS (SEQ ID NO:54) |
| 66 | HEC-G71 | YGEGT FTSDY SIYLD KKAQR EFIDW LLAG GPSSGAPPPS (SEQ ID NO:55) |
| 67 | HEC-G72 | YGEGT FTSDY SIYLD KKAQR DFIDW LLAG GPSSGAPPPS (SEQ ID NO:56) |
| 68 | HEC-G73 | YGEGT FTSDY SIYLD KKAQR AFIDW LLAG GPSSGAPPPS (SEQ ID NO:57) |
| 69 | HEC-G74 | YGEGT FTSDY SIYLD KKAQR DFIEW LIAQ GPSSGAPPPS (SEQ ID NO:58) |
| 70 | HEC-G75 | YGEGT FTSDY SIYLD KKAQR DFVEW LIAQ GPSSGAPPPS (SEQ ID NO:59) |
| 71 | HEC-G76 | YGEGT FTSDY SIYLD KKAQR DFVQW LLAG GPSSGAPPPS (SEQ ID NO:60) |
| 72 | HEC-G77 | YGEGT FTSDY SIILD KKAQR DFVEW LLAG GPSSGAPPPS (SEQ ID NO:61) |
| 73 | HEC-G78 | YGEGT FTSDY SIALD KKAQR DFIEW LIAG GPSSGAPPPS (SEQ ID NO:62) |
| 74 | HEC-G79 | YGEGT FTSDY SIYLD KKAQR DFIEW LLAG (SEQ ID NO:63) |
| 75 | HEC-G80 | YGEGT FTSDY SIYLD KKAQR DFIEY LLAG (SEQ ID NO:64) |
| 76 | HEC-G81 | YGEGT FTSDY SIALD KQAQR DFVEW LIAG GPSSGAPPPS (SEQ ID NO:65) |
| 77 | HEC-G82 | YGEGT FTSDY SIYLD KQAQR DFVEW LLAQ GPSSGAPPPS (SEQ ID NO:66) |
| 78 | HEC-G83 | YGEGT FTSDY SIYLD KIAQR DFVEW LIAG GPSSGAPPPS (SEQ ID NO:67) |
| 79 | HEC-G84 | YGEGT FTSDY SIYLD KQHQR DFVEW LIAG GPSSGAPPPS (SEQ ID NO:68) |
| 80 | HEC-G85 | YGEGT FTSDY SIYLD KKAQR DFINW LIAG GPSSGAPPPS (SEQ ID NO:69) |
| 81 | HEC-G86 | YGEGT FTSDY SIYLD KKHQR DFIEW LIAG GPSSGAPPPS (SEQ ID NO:70) |
| 82 | HEC-G87 | YGEGT FTSDY SIYLD KIAQR DFIEW LIAG GPSSGAPPPS (SEQ ID NO:71) |
| 83 | HEC-G88 | YGEGT FTSDY SIYLD KKAQQ DFIEW LIAG GPSSGAPPPS (SEQ ID NO:72) |
| 84 | HEC-G89 | YGEGT FTSDY SIALD KQAQR DFVEW LLAG GPSSGAPPPS (SEQ ID NO:73) |
| 85 | HEC-G90 | YGEGT FTSDY SIYLD KQAQQ DFVEW LLAG GPSSGAPPPS (SEQ ID NO:74) |
| 86 | HEC-G91 | YGEGT FTSDY SIYLD KIAQR DFVEW LLAG GPSSGAPPPS (SEQ ID NO:75) |
| 87 | HEC-G92 | YGEGT FTSDY SIYLD KQAQR DFVNW LLAG GPSSGAPPPS (SEQ ID NO:76) |
| 88 | HEC-G93 | YGEGT FTSDY SIYLD KIAQQ AFVEW LLAG GPSSGAPPPS (SEQ ID NO:77) |
| 89 | HEC-G94 | YGEGT FTSDY SIYMD KLAQQ AFVEW LLAG GPSSGAPPPS (SEQ ID NO:78) |
| 90 | HEC-G95 | YGEGT FTSDY SIYLD KLAQQ AFVNW LLAG GPSSGAPPPS (SEQ ID NO:79) |
| 91 | HEC-G96 | YSEGT FTSDY SIYLD KIAQR DFVEW LLAG GPSSGAPPPS (SEQ ID NO:80) |
| 92 | HEC-G97 | YSEGT FTSDY SIALD KKAQR DFVEW LLAG GPSSGAPPPS (SEQ ID NO:81) |
| 93 | HEC-G98 | YSEGT FTSDY SIYLD KKAQQ DFVEW LLAG GPSSGAPPPS (SEQ ID NO:82) |
| 94 | HEC-G99 | YGEGT FTSDY SIALD KKAQR DFIDW LLAG GPSSGAPPPS (SEQ ID NO:83) |
| 95 | HEC-G100 | YGEGT FTSDY SIYLD KKAQR DFINW LLAG GPSSGAPPPS (SEQ ID NO:84) |
| 96 | HEC-G101 | YGEGT FTSDY SIYLD KKAQQ DFIDW LLAG GPSSGAPPPS (SEQ ID NO:85) |
| 97 | HEC-G102 | YGEGT FTSDY SIYLD KKAQR DFINW LIAQ GPSSGAPPPS (SEQ ID NO:86) |
| 98 | HEC-G103 | YGEGT FTSDY SIALD KKAQR DFIEW LIAQ GPSSGAPPPS (SEQ ID NO:87) |
| 99 | HEC-G104 | YGEGT FTSDY SIYLD KKAQQ DFIEW LIAQ GPSSGAPPPS (SEQ ID NO:88) |
| 100 | HEC-G105 | YGEGT FTSDY SIYLD KIAQR DFIEW LIAQ GPSSGAPPPS (SEQ ID NO:89) |
| 101 | HEC-G106 | YGEGT FTSDY SILLD KKAQR DFVEW LLAG GPSSGAPPPS (SEQ ID NO:90) |
| 102 | HEC-G107 | YGEGT FTSDY SIYLD KKAQH DFVEW LLAG GPSSGAPPPS (SEQ ID NO:91) |
| 107 | HEC-G112 | YGEGT FTSDY SIYLD KQAQH AFIEW LIAG GPSSGAPPPS (SEQ ID NO:92) |
| 108 | HEC-G113 | HSEGT FTSDY SILLD KIAQQ DFVEW LLAG GPSSGAPPPS (SEQ ID NO:93) |
| 109 | HEC-G114 | HSEGT FTSDY SILLD KIAQH DFIEW LIAG GPSSGAPPPS (SEQ ID NO:94) |
| 110 | HEC-G115 | YSEGT FTSDY SILLD KIAQR EFIEW LLAG GPSSGAPPPS (SEQ ID NO:95) |
| 111 | HEC-G116 | HSEGT FTSDY SILLD KIAQR EFIEW LLAG GPSSGAPPPS (SEQ ID NO:96) |
| 112 | HEC-G117 | HSEGT FTSDY SILLD KIAQR EFIEW LLEG GPSSGAPPPS (SEQ ID NO:97) |
| 113 | HEC-G118 | HSEGT FTSDY SILLD KIAQK EFIEW LLAG GPSSGAPPPS (SEQ ID NO:98) |
| 114 | HEC-G119 | YSEGT FTSDY SILLD KIAQK EFIEW LLAG GPSSGAPPPS (SEQ ID NO:99) |
| 115 | HEC-G120 | HSEGT FTSDY SILLD KIAQK EFIEW LLEG GPSSGAPPPS (SEQ ID NO:100) |
| 116 | HEC-G121 | YSEGT FTSDY SILLD KIAQRAFIEW LLAG GPSSGAPPPS (SEQ ID NO:101) |
| 117 | HEC-G122 | YSEGT FTSDY SILLD KIAQR EFIEW LIAG GPSSGAPPPS (SEQ ID NO:102) |
| 118 | HEC-G123 | YSEGT FTSDY SILLD KIAQR EFVQW LLAG GPSSGAPPPS (SEQ ID NO:103) |
| 119 | HEC-G124 | YSEGT FTSDY SILLD KIAQR EFVQW LIAG GPSSGAPPPS (SEQ ID NO:104) |
| 120 | HEC-G125 | YSEGT FTSDY SILLD KIAQR AFVQW LIAG GPSSGAPPPS (SEQ ID NO:105) |
| 121 | HEC-G126 | YGEGT FTSDY SILLD KIAQR EFIEW LLAG GPSSGAPPPS (SEQ ID NO:106) |
| 122 | HEC-G127 | YSEGT FTSDY SILLD KKAQR DFIEW LLAG GPSSGAPPPS (SEQ ID NO:107) |
| 123 | HEC-G128 | YGEGT FTSDY SILLD KKAQR DFIEW LLAG GPSSGAPPPS (SEQ ID NO:108) |
| 124 | HEC-G129 | YSEGT FTSDY SILLD KKAQR DFIEW LIAG GPSSGAPPPS (SEQ ID NO:109) |
| 125 | HEC-G130 | YSEGT FTSDY SILLD KKAQR DFVQW LIAG GPSSGAPPPS (SEQ ID NO:110) |
| 126 | HEC-G131 | YSEGT FTSDY SILLD KIAQR DFVEW LIAG GPSSGAPPPS (SEQ ID NO:111) |
| 127 | HEC-G132 | YGEGT FTSDY SILLD KIAQR DFVEW LIAG GPSSGAPPPS (SEQ ID NO:112) |
| 128 | HEC-G133 | YSEGT FTSDY SILLD KIAQQ DFVEW LLAG GPSSGAPPPS (SEQ ID NO:117) |
| 129 | HEC-G134 | YSEGT FTSDY SILLD KIAQQ EFIEW LLAG GPSSGAPPPS (SEQ ID NO:118) |
| 130 | HEC-G135 | YSEGT FTSDY SILLD KIAQQ EFIEW LIAG GPSSGAPPPS (SEQ ID NO:119) |
| 131 | HEC-G136 | YSEGT FTSDY SILLD KKAQQ DFIEW LLAG GPSSGAPPPS (SEQ ID NO:120) |
| 132 | HEC-G137 | YSEGT FTSDY SILLD KKAQQ DFIEW LIAG GPSSGAPPPS (SEQ ID NO:121) |

### Example 4 Vector transfection and expression in cells

In this example, CHO-S cells were resuscitated and subcultured, the cell density was diluted to about 6*10⁶ cells/mL. ExpiCHO FectamineTM CHO Transfection Kit (ThermoFisher Scientific) was used for transfection. Taking the 50 mL expression system as an example, the specific experiment operation was as follows:
1) The plasmid was diluted by 2 mL of OptiPRO^{™} SFM Complexation Medium, the plasmid needed to be filtered and sterilized. The final concentration was 1ug/mL;
2) 1.84 mL OptiPRO^{™} SFM Complexation Medium was added to 160 µL ExpiFectamine^{™} CHO Reagent;
3) The solutions in steps 1 and 2 were mixed evenly and added into the cells, marked respectively and the mixture was placed in a shaker, and incubated at 37°C, 8% CO₂, 140rpm for 18-22h;
4) After the culturing, 300 µL of ExpiFectamine^{™} CHO Enhancer and 12 mL of ExpiCHO^{™} Feed were added respectively to the transfected cells;
5) The transfected cells obtained in step 4) were placed on a shaker at 37°C, 8% CO2, 140 rpm. After culturing for six days, the cell fermentation broth was collected for subsequent purification.

### Example 5 Purification and Identification of Fusion Protein

The cell culture medium was centrifuged to collect the supernatant, and filtered with a 0.22 µm filter to remove residual cell debris. The collected cell culture medium was purified using a Protein A chromatography column to collect the target peak, and then further purified using anion exchange chromatography. The protein was finally eluted and collected with 0.02M PBS. The specific steps are as follows:
1) The cell fermentation broth was collected by a 50mL centrifuge tube and centrifuged at 1000rpm for 10 min at low speed;
2) At the same time, the column and purification system were rinsed with ultrapure water for 5-10 column volumes;
3) A 0.45 µm ultrafiltration membrane was used to filter the supernatant after centrifugation to remove cell sediment;
4) The column was washed with 0.02M PBS buffer for 5-10 column volumes and the column was equilibrated;
5) The filtered supernatant was flowed through the column until all the samples flow through the column;
6) Then the column was rinsed with 0.02M PBS buffer for 5-10 column volumes until the sample baseline reached "0";
7) Then the sample was eluted using 0.1M acetic acid-sodium acetate solution into a 50mL centrifuge tube containing 2.5mLof a pH 8.0 Tris-HCl solution;
8) The column was rinsed with 0.1M NaOH solution (5-10 column volumes), then the packing was regenerated, and some impurity proteins were removed in the column;
9) The column was rinsed with ultrapure water for 5-10 column volumes;
10) The above operations 4) ~ 9) were repeated until all samples were loaded;
11) The system was rinsed with 20% ethanol, finally the column was stored in ethanol, and then the column was removed;
12) The protein A prepacked column was removed from the purifier and replaced it with the anion exchange chromatography Q column;
13) The column and purification system were rinsed with ultrapure water for 5-10 column volumes;
14) The column was washed with 0.02M PB buffer for 5-10 column volumes for equilibrium;
15) The conductivity of the initially pure sample obtained in the above operation (7) was diluted to below 5ms/cm and then the sample was loaded;
16) Then the column was washed with 0.02M PB buffer for 5-10 column volumes;
17) The protein sample was eluted with 0.02M PBS buffer;
18) The sample was eluted with 1.5M NaCl solution to remove the pigment in the column;
19) The column was rinsed with 0.1M NaOH solution (5-10 column volumes) to remove some impurity proteins in the column;
20) The column was rinsed with ultrapure water for 5-10 column volumes;
21) The above operations 14) ~ 20) were repeated until all samples were loaded;
22) The system and the column were rinsed with 20% ethanol until only ethanol was remained in the column.

The samples were quantified using a micronucleic acid protein analyzer (NanoDrop 2000/2000c Spectrophotometer), and then detected by 12% SDS-PAGE electrophoresis, and the electrophoresis results showed a single band.

### Example 6 Determination of in vitro activity of fusion protein

HEK293 cells expressing GLP-1R or GIPR were treated the fusion protein prepared by expressing, human GLP-1 (purchased) and GIP (purchased), respectively, the specific operations are as follows:
1) The genes GIPR and GLP-1R were optimized and routinely synthesized at Genewiz, and the genes were cloned into the vector pUC57-Amp to prepare mini-scale recombinant plasmid DNA and puncture bacteria containing the recombinant plasmid;
2) pUC57-GIPR recombinant plasmid DNA was double-digested with H*ind*III and *EcoR* I, and pUC57-GLP-1R was double-digested with H*in*dIII and X*ho*I. The digested product was electrophoresed on 1% agarose gel and the target band was cut out with a clean blade, then a gel recovery kit was used to recover the target fragment. The specific experimental operations are carried out according to the kit instructions;
3) The target fragment enzymatic digestion recovery product and vector plasmid pcDNA3.1 fragment were connected through T4 ligase and transformed into DH5α competent cells, then single colonies were isolated by spreading on plates, and the transformants were selected and expanded for enzyme digestion verification and sequencing verification.
4) 200mL of the bacterial liquid obtained in inoculation step 3) and verified by sequencing to contain the fusion protein of the target product was used for plasmid extraction. The kit used was PureLink HiPure Plasmid Maxiprep Kit, and according to the instructions. After the plasmid was verified to be correct by PCR and enzyme digestion, pvul restriction enzyme was used for linearization. Finally, the ethanol precipitation method was used to recover the plasmid.
5) The host cells were HEK293, one day before transfection, the cells were spread into a 6-well plate at a density of 2x10^6 cells/well, and the addition volume was 1mL/well. The recovered linearized plasmid was transfected into HEK293 cells using Lipofectamine 3000 transfection method, then G418 was added to screen to obtain mixed strains, single clones were obtained by limiting dilution and isolation, and the activity was tested and verified.

The cAMP produced by the recipient cells was detected by a cAMP detection kit (Cisbio, 62AM6PEC) according to the steps described in the operating instructions. The specific steps are as follows:
1) Preparation of Assay buffer: To the complete culture medium (DMEM medium + 10% FBS) was added 4/1000 of 500 mM IBMX stock solution, cAMP-d2 working solution and anti-cAMP-crytate working solution were prepared according to the kit instructions;
2) The test sample and control sample human GLP-1 (purchased) and GIP (purchased) were diluted to a stock solution with an initial concentration of 500 nM, and then 20 µL of which was added to 80 µL Assay buffer (diluted 5 times) in a gradient step-by-step dilution, a total of 8 compound gradients including stock solution were obtained;
3) Preparation of cell suspension: The cells HEK293-GLP-1R and HEK293-GIPR were taken out from a liquid nitrogen tank, and placed in a 37°C water bath immediately. If the mixture was not completely melted within 1.5 min, the cells were added dropwise to a 15mL centrifuge tube containing 8mL warm culture medium on a clean bench, then centrifuged at 900rpm for 5 min, the supernatant was discarded, and the cells were re-suspended with 1mL complete culture medium (15 times by pipetting). 20µL of the suspension was taken immediately and mixed with an equal volume of trypan blue, the cells were diluted to 4×10^5 cells/mL after taking 20 µL to count the number of viable cells.
4) A 384-well plate was divided into GLP-1R cell area and GIPR cell area, cell suspension was added to wells in the corresponding area using a 12-channel adjustable dispenser with 5 µL per well, and then the serial dilutions of the test substance and positive control substance were added into the 384-well plate corresponding to the cells using a 12-channel adjustable dispenser with 5 µL per well (samples of the same concentration were replicated in 2 parallel wells); negative control: 10 µL assay buffer /well, each 384-well plate was set 3 wells and covered with a white sealing film, and placed in a 37°C constant temperature incubator, then taken out after 0.5 h;
5) The cAMP-d2 working solution and anti-cAMP-crytate working solution were diluted 20-fold with lysis buffer in the Hi-range kit before use, and then mixed evenly according to 1: 1 to prepare the cAMP detection reagent mixture, cAMP detection reagent mixture was added with 10 µL/well of the sample group, and 5 µL of lysis buffer and 5 µL of diluted anti-cAMP-crytate working solution were added to each well of the negative control, then the plate was covered with a white lid, and placed at room temperature in the dark for 1 h.
6) The fluorescence values at 665nm and 620nm were detected by a multifunctional microplate reader;
7) Using this to establish a dose-response curve, then EC50 value was calculated, and compared each other.

The specific results are shown in Table 5, in which the fusion proteins shown in G04, G05, G12, G27, G28, G30, G42, G49-G51, G58, G62, G108-G111 showed no agonistic activity, and other fusion proteins were detected with agonistic activity, among which, G06, G20, G23, G34, G35, G37, G38, G48, G66, G67, G72, G74, G99, G101, G113, G115, G121-G127, G130-G132 had strong activity in vitro.

**Table 5:**

| No. | Name of the fusion protein | EC50 (nM) of the fusion protein | |
|---|---|---|---|
| | | GLP-1R | GIPR |
| | Human GLP-1 | 0.053 | N/A |
| | Human GIP | N/A | 0.121 |
| 1 | HEC-G01 | 0.582 | 10.184 |
| 2 | HEC-G02 | 0.451 | 16.143 |
| 3 | HEC-G03 | 4.054 | 2.417 |
| 4 | HEC-G04 | N/A | N/A |
| 5 | HEC-G05 | N/A | N/A |
| 6 | HEC-G06 | 1.594 | 0.585 |
| 7 | HEC-G07 | 1.057 | 4.835 |
| 8 | HEC-G08 | 1.578 | 15.835 |
| 9 | HEC-G09 | 0.165 | 18.750 |
| 10 | HEC-G10 | 0.264 | 15.453 |
| 11 | HEC-G11 | 0.497 | 11.235 |
| 12 | HEC-G12 | N/A | N/A |
| 13 | HEC-G13 | 0.685 | 1.728 |
| 14 | HEC-G14 | 0.114 | 10.054 |
| 15 | HEC-G20 | 0.262 | 0.197 |
| 16 | HEC-G21 | 0.134 | 22.773 |
| 17 | HEC-G22 | 0.165 | 2.585 |
| 18 | HEC-G23 | 0.054 | 0.301 |
| 19 | HEC-G24 | 59.117 | 0.675 |
| 20 | HEC-G25 | 3.751 | 0.134 |
| 21 | HEC-G26 | 624470.711 | 0.064 |
| 22 | HEC-G27 | N/A | N/A |
| 23 | HEC-G28 | 1.971 | 0.295 |
| 24 | HEC-G29 | 2.731 | 0.163 |
| 25 | HEC-G30 | N/A | N/A |
| 26 | HEC-G31 | 2.557 | 2.852 |
| 27 | HEC-G32 | 1.864 | 0.321 |
| 28 | HEC-G33 | 0.391 | 2.234 |
| 29 | HEC-G34 | 0.354 | 0.275 |
| 30 | HEC-G35 | 2.862 | 1.241 |
| 31 | HEC-G36 | N/A | N/A |
| 32 | HEC-G37 | 0.745 | 0.563 |
| 33 | HEC-G38 | 12.531 | 0.589 |
| 34 | HEC-G39 | 0.675 | 1.342 |
| 35 | HEC-G40 | 0.061 | 10.876 |
| 36 | HEC-G41 | 1.574 | 6.022 |
| 37 | HEC-G42 | N/A | N/A |
| 38 | HEC-G43 | 0.961 | 4.992 |
| 39 | HEC-G44 | 2.552 | 0.171 |
| 40 | HEC-G45 | 0.140 | 2.416 |
| 41 | HEC-G46 | 0.285 | 0.297 |
| 42 | HEC-G47 | 0.995 | 0.651 |
| 43 | HEC-G48 | 1.162 | 0.091 |
| 44 | HEC-G49 | N/A | N/A |
| 45 | HEC-G50 | N/A | N/A |
| 46 | HEC-G51 | N/A | N/A |
| 47 | HEC-G52 | 2.723 | 1.214 |
| 48 | HEC-G53 | 0.772 | 0.518 |
| 49 | HEC-G54 | 0.121 | 1.042 |
| 50 | HEC-G55 | 3.980 | 0.278 |
| 51 | HEC-G56 | 746490.000 | 0.154 |
| 52 | HEC-G57 | 17.462 | 9.431 |
| 53 | HEC-G58 | N/A | N/A |
| 54 | HEC-G59 | 0.844 | 6.093 |
| 55 | HEC-G60 | 8.751 | 3.052 |
| 56 | HEC-G61 | 7.584 | 1.147 |
| 57 | HEC-G62 | N/A | N/A |
| 58 | HEC-G63 | 39.051 | 17.714 |
| 59 | HEC-G64 | 6.324 | 0.875 |
| 60 | HEC-G65 | 1.577 | 3.782 |
| 61 | HEC-G66 | 7.031 | 0.324 |
| 62 | HEC-G67 | 1.137 | 0.485 |
| 63 | HEC-G68 | 0.272 | 10.171 |
| 64 | HEC-G69 | 38.931 | 0.332 |
| 65 | HEC-G70 | 0.684 | 3.885 |
| 66 | HEC-G71 | 0.252 | 1.041 |
| 67 | HEC-G72 | 0.281 | 0.372 |
| 68 | HEC-G73 | 0.155 | 5.931 |
| 69 | HEC-G74 | 1.997 | 0.552 |
| 70 | HEC-G75 | 0.876 | 1.825 |
| 71 | HEC-G76 | 0.714 | 5.012 |
| 72 | HEC-G77 | 3.884 | 4.837 |
| 73 | HEC-G78 | 0.226 | 1.077 |
| 74 | HEC-G79 | 3.245 | 0.779 |
| 75 | HEC-G80 | 1.252 | 3.184 |
| 76 | HEC-G81 | 0.427 | 0.922 |
| 77 | HEC-G82 | 6.534 | 0.172 |
| 78 | HEC-G83 | 0.125 | 0.732 |
| 79 | HEC-G84 | 6.034 | 8.970 |
| 80 | HEC-G85 | 0.045 | 0.326 |
| 81 | HEC-G86 | 0.651 | 5.495 |
| 82 | HEC-G87 | 0.127 | 0.266 |
| 83 | HEC-G88 | 0.155 | 0.258 |
| 84 | HEC-G89 | 2.662 | 0.288 |
| 85 | HEC-G90 | 105.400 | 0.945 |
| 86 | HEC-G91 | 5.697 | 2.155 |
| 87 | HEC-G92 | 1.637 | 0.677 |
| 88 | HEC-G93 | 8.592 | 0.437 |
| 89 | HEC-G94 | 8.284 | 0.255 |
| 90 | HEC-G95 | 16.130 | 2.222 |
| 91 | HEC-G96 | 3.664 | 0.547 |
| 92 | HEC-G97 | 4.382 | 0.825 |
| 93 | HEC-G98 | 18.094 | 0.321 |
| 94 | HEC-G99 | 1.942 | 1.142 |
| 95 | HEC-G100 | 0.211 | 0.464 |
| 96 | HEC-G101 | 7.474 | 0.177 |
| 97 | HEC-G102 | 0.378 | 0.545 |
| 98 | HEC-G103 | 4.925 | 1.224 |
| 99 | HEC-G104 | 14.932 | 0.762 |
| 100 | HEC-G105 | 6.947 | 1.922 |
| 101 | HEC-G106 | 0.945 | 0.477 |
| 102 | HEC-G107 | 6.473 | 0.235 |
| 103 | HEC-G108 | N/A | N/A |
| 104 | HEC-G109 | N/A | N/A |
| 105 | HEC-G110 | N/A | N/A |
| 106 | HEC-G111 | N/A | N/A |
| 107 | HEC-G112 | 0.268 | 0.564 |
| 108 | HEC-G113 | 0.395 | 0.066 |
| 109 | HEC-G114 | 0.047 | 0.034 |
| 110 | HEC-G115 | 1.077 | 0.042 |
| 111 | HEC-G116 | 0.042 | 0.034 |
| 112 | HEC-G117 | 0.047 | 0.032 |
| 113 | HEC-G118 | 0.076 | 0.039 |
| 114 | HEC-G119 | 8.737 | 0.025 |
| 115 | HEC-G120 | 0.092 | 0.071 |
| 116 | HEC-G121 | 0.417 | 0.036 |
| 117 | HEC-G122 | 0.155 | 0.023 |
| 118 | HEC-G123 | 1.046 | 0.012 |
| 119 | HEC-G124 | 0.168 | 0.024 |
| 120 | HEC-G125 | 0.354 | 0.057 |
| 121 | HEC-G126 | 0.965 | 0.015 |
| 122 | HEC-G127 | 0.477 | 0.042 |
| 123 | HEC-G128 | 0.394 | 0.112 |
| 124 | HEC-G129 | 0.066 | 0.059 |
| 125 | HEC-G130 | 0.052 | 0.318 |
| 126 | HEC-G131 | 0.277 | 0.028 |
| 127 | HEC-G132 | 0.285 | 0.027 |
| 128 | HEC-G133 | 63.245 | 0.006 |
| 129 | HEC-G134 | 16.277 | 0.004 |
| 130 | HEC-G135 | 1.268 | 0.033 |
| 131 | HEC-G136 | 8.223 | 0.018 |
| 132 | HEC-G137 | 0.357 | 0.021 |

### Example 7 Evaluation of Glucose tolerance

This example evaluates the effect of HEC-G123, HEC-G128, HEC-G131, HEC-G132 on glucose tolerance in normal C57BL/6 mice.

Experimental methods: Normal C57BL/6 mice were randomly divided into 6 groups (Vehicle group, Dulaglutide group, HEC-G123 group, HEC-G128 group, HEC-G131 group, HEC-G132 group) according to blood glucose and body weight, with 8 mice in each group. For the groups of Dulaglutide, HEC-G123, HEC-G128, HEC-G131 and HEC-G132, the corresponding drug was injected subcutaneously into the mice at a dose of 3 nmol/kg, and for the control group, the corresponding vehicle was injected subcutaneously.

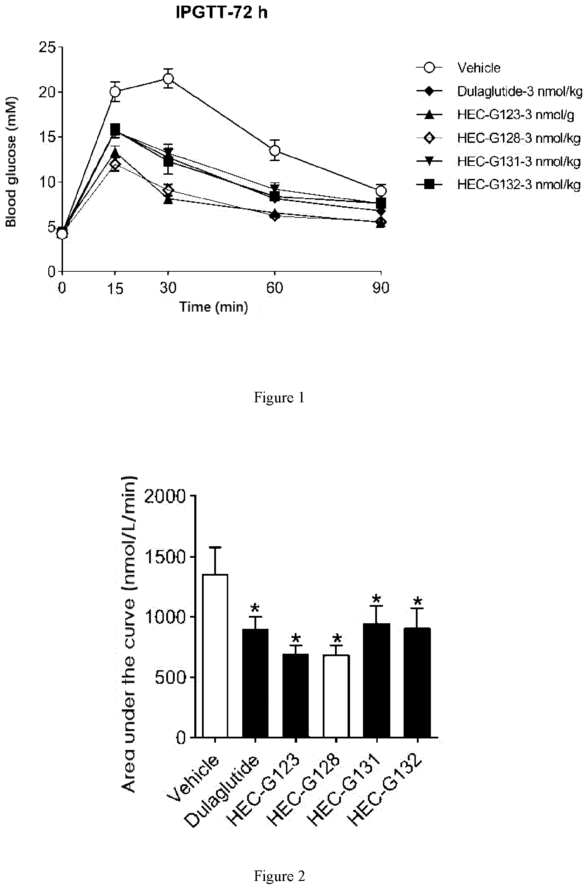

After 60 h of single administration, the mice were fasted for 12 h and drank water freely. Blood was collected from the tail vein to measure the basal blood glucose value of each group, then 2 g/kg of glucose solution was injected intraperitoneally, and blood glucose was measured at 15, 30, 60, and 0 min after glucose administration. According to the blood glucose values measured at different time points, the blood glucose concentration-time curve was plotted, and the AUC_{0~ 90min} of each dose group was calculated, and the experimental results are shown in Table 6 and Figures 1-2.

**Table 6: Gluco·se tolerance effect of HEC-G123, HEC-G128, HEC-G131 and HEC-G132 in normal C57 mice after a single dose for 72 h**

| Group | Blood glucose value (mM) | | | | | AUC_{0-90 min} |
|---|---|---|---|---|---|---|
| | 0 min | 15 min | 30 min | 60 min | 90 min | |
| Vehicle | 4.2±0.4 | 20.1±3.2 | 21.5±3.1 | 13.5±3.2 | 9.0±2.1 | 1,355.8±220.8 |
| Dulaglutide | 4.4±0.8 | 15.7±2.2 | 12.7±2.4 | 8.1±1.0 | 6.8±0.6 | 899.0±105.4^{a} |
| HEC-G123 | 4.6±0.7 | 13.3±1.9 | 8.1±1.1 | 6.6±0.6 | 5.5±0.4 | 696.8±69.4^{a} |
| HEC-G128 | 4.2±1.1 | 11.9±2.0 | 9.1±1.7 | 6.2±0.9 | 5.6±0.6 | 686.1±79.5^{a} |
| HEC-G131 | 4.0±0.8 | 15.6±1.9 | 13.2±2.8 | 9.2±2.0 | 7.6±1.4 | 950.4±140.4^{a} |
| HEC-G132 | 4.3±0.9 | 15.7±2.2 | 12.3±4.1 | 8.4±1.2 | 7.6±0.7 | 911.1±160.5^{a} |

| | | | | | | |
|---|---|---|---|---|---|---|
| [0099] Note: The lowercase letters of the shoulder mark in the same column indicate significant differences (P<0.05) [00100] Conclusion: HEC-G123, HEC-G128, HEC-G131 and HEC-G132 significantly reduced the blood glucose level of normal C57 mice after a single dose for 72 h. Compared with the positive control Dulaglutide, HEC-G123 and HEC-G128 had a more significant effect on improving glucose tolerance, while HEC-G131 and HEC-G132 had no significant difference in improving glucose tolerance. | | | | | | |

### Example 8 Evaluation of Glucose tolerance

This example evaluates the effect of HEC-G113, HEC-G122, HEC-G126, HEC-G127 on glucose tolerance in normal C57BL/6 mice.

Experimental methods: Normal C57BL/6 mice were randomly divided into 6 groups (Vehicle group, Dulaglutide group, HEC-G113 group, HEC-G122 group, HEC-G126 group, HEC-G127 group) according to blood glucose and body weight, with 8 mice in each group. For the groups of Dulaglutide (Dulaglutide purchased from Eli Lilly, lot number was D256504), HEC-G113, HEC-G122, HEC-G126 and HEC-G127, the corresponding drug was injected subcutaneously into the mice at a dose of 3 nmol/kg, and for the control group, the corresponding vehicle was injected subcutaneously.

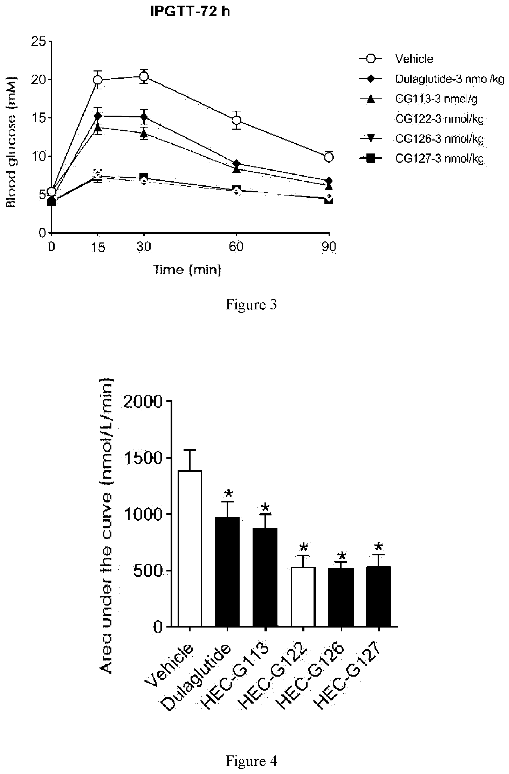

After 60 h of single administration, the mice were fasted for 12 h and drank water freely. Blood was collected from the tail vein to measure the basal blood glucose value of each group, then 2 g/kg of glucose solution was injected intraperitoneally, and blood glucose was measured at 15, 30, 60, 90 and 0 min after glucose administration. According to the blood glucose values measured at different time points, the blood glucose concentration-time curve was plotted, and the AUC_{0∼ 90min} of each dose group was calculated, and the experimental results are shown in Table 7 and Figures 3-4.

**Table 7: Glucose tolerance effect of HEC-G113, HEC-G122, HEC-G126 and HEC-G127 in normal C57 mice after a single dose for 72 h**

| Group | Blood glucose value (mM) | | | | | AUC_{0-90 min} |
|---|---|---|---|---|---|---|
| | 0 min | 15 min | 30 min | 60 min | 90 min | |
| Vehicle | 5.4±0.8 | 20.0±3.4 | 20.4±2.7 | 14.7±3.4 | 9.9±2.1 | 1,387.5±178.3 |
| Dulaglutide | 4.3±0.4 | 15.3±3.1 | 15.1±2.7 | 9.1±1.3 | 6.8±0.9 | 976.2±135.6^{a} |
| HEC-G113 | 5.4±0.7 | 13.8±2.7 | 13.0±2.3 | 8.3±1.0 | 6.2±0.7 | 882.8±115.2^{a} |
| HEC-G122 | 4.5±0.4 | 7.8±1.9 | 6.6±1.7 | 5.3±1.1 | 4.8±0.7 | 531.5±103.1^{a} |
| HEC-G126 | 4.0±0.4 | 7.3±1.0 | 6.7±0.7 | 5.4±0.7 | 4.5±1.0 | 520.1±54.2^{a} |
| HEC-G127 | 4.1±0.4 | 7.4±2.2 | 7.1±1.7 | 5.6±0.9 | 4.4±0.7 | 536.3±106.4^{a} |

| | | | | | | |
|---|---|---|---|---|---|---|
| Note: The lowercase letters of the shoulder mark in the same column indicate significant differences (P<0.05) Conclusion: HEC-G113, HEC-G122, HEC-G126 and HEC-G127 significantly reduced the blood glucose levels of normal C57 mice after a single dose for 72 h. The effect of each group in improving glucose tolerance was better than that of the positive control Dulaglutide. | | | | | | |

### Example 9 In vivo efficacy evaluation of the 9 db/db mouse model

This example evaluates the glucose effect of HEC-G20 on a db/db mouse model.

Methods: 7-8 week old db/db mice were randomly divided into 3 groups (Model group, Semaglutide group, HEC-G20 group) according to blood glucose and body weight, with 9 mice in each group. For the groups of Semaglutide (Semaglutide was purchased from Novo Nordisk, lot number was JP52092) and HEC-G20, the corresponding drug was administered subcutaneously into the mice at a dose of 10 nmol/kg for each group, and for the Model group, the corresponding vehicle was injected subcutaneously. The Semaglutide group was dosed once a day, and the HEC-G20 group was dosed twice a week for a total of 4 weeks, and animal blood glucose was tested before each dose.

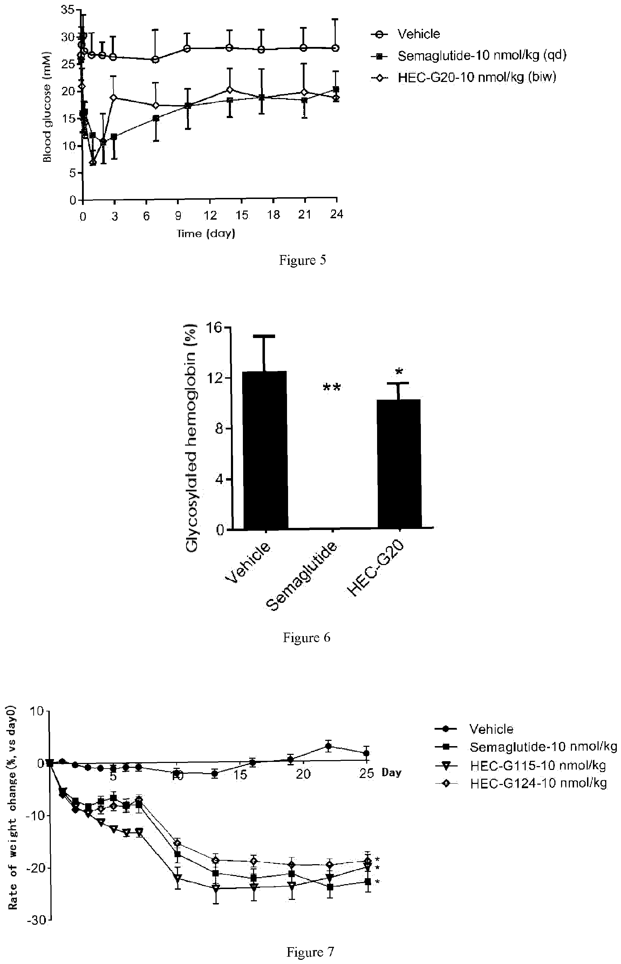

Experimental results: The HEC-G20 group was able to significantly reduce blood glucose after administration, and the blood glucose value reached the lowest level at 7 h, the effect was similar to that of the positive control Semaglutide at the same dose. Compared with Model group, the blood glucose value of mice in the long-term repeated dose of HEC-G20 group was significantly lower and remained stable for a long time, and its hypoglycemic effect was similar to that of the Semaglutide group, as shown in Table 8 and Figures 5-6.

**Table 8: Effect of long-term administration of HEC-G20 on blood glucose in db/db mice**

| Group | Random blood glucose (mM) | | | | | | | | | | |
|---|---|---|---|---|---|---|---|---|---|---|---|
| | D0 | 1.5 h | 5 h | 7 h | D3 | D7 | D10 | D14 | D17 | D21 | D24 |
| Model | 26.6 ±3.0 | 28.6± 3.3 | 30.3± 3.8 | 27.4± 3.3 | 26.3± 3.8 | 25.7± 5.4 | 27.6± 2.8 | 27.7± 3.2 | 27.3± 3.6 | 27.6± 3.7 | 27.5± 5.3 |
| Semaglutide | 25.7 ±3.7 | 15.9± 3.4 | 15.4± 4.0 | 16.3± 4.2 | 11.6± 4.1 | 14.9± 4.2 | 17.1± 4.2 | 18.1± 3.1 | 18.5± 3.0 | 18.0± 3.3 | 19.9± 2.2^{a} |
| HEC-G20 | 26.0 ±4.7 | 20.9± 3.3 | 14.8± 3.4 | 14.2± 3.8 | 18.8± 4.0 | 17.3± 4.2 | 17.2± 3.0 | 20.0± 4.0 | 18.5± 5.2 | 19.4± 5.2 | 18.4± 4.8^{a} |

| | | | | | | | | | | | |
|---|---|---|---|---|---|---|---|---|---|---|---|
| Note: The lowercase letters of the shoulder mark in the same column indicate significant differences (P<0.05) Conclusion: Long-term administration of HEC-G20 can significantly improve blood glucose levels in type II diabetes db/db mice. | | | | | | | | | | | |

### Example 10 In vivo efficacy evaluation of the DIO model

In this example, the effects of long-term repeated administration of HEC-G115 and HEC-G124 on the body weight and feeding of obese mice in the DIO model were evaluated.

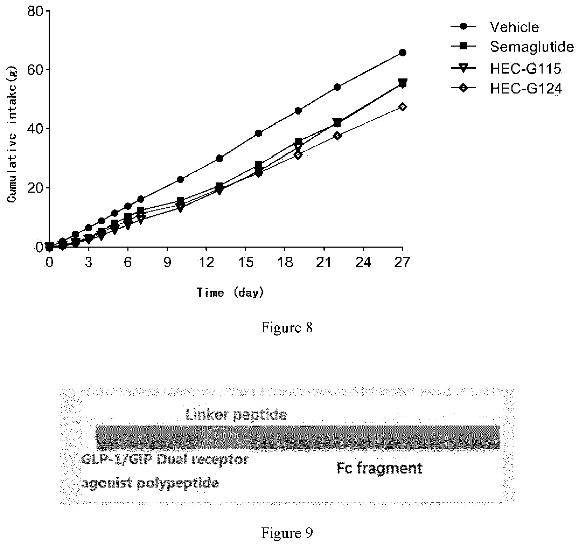

Experimental methods: C57BL6 mice were randomly divided into normal group NFD and model group HFD at the fifth week of age, and the normal group was fed with ordinary maintenance diet, while the model group was fed with high-fat diet D12492. Changes in body weight and food intake in mice were monitored every 3 weeks. After 16 weeks of feeding, the body weight of mice in the model group and the normal group were (47.9±3.4) g and (29.6±1.5) g, respectively, and the difference between the two groups was statistically significant. The normal group was divided into the control group, and the model group mice that were successfully modeled were divided into Vehicle group, Semaglutide group, HEC-G115 group and HEC-G124 group, with 10 mice in each group. For the groups of Semaglutide, HEC-G115 and HEC-G124, the corresponding drug was injected subcutaneously in each group, and PBS was injected subcutaneously in the Vehicle group. The first administration was observed for 7 days, and then every 3 days in each group, at a dose of 10 nmol/kg. Weight and food intake were measured before each dose. After 3 weeks of administration, an intraperitoneal glucose tolerance test was performed. After 72 h of the last administration, samples were collected, liver weight was recorded, and liver pathological status and blood biochemical indexes were detected in each group. The test results are shown in Tables 9-12 and Figures 7-8.

**Table 9: Effect of long-term administration of HEC-G115 and HEC-G124 on the body weight of DIO mice**

| Group | Weight growth rate (%) | | | | | | |
|---|---|---|---|---|---|---|---|
| | D0 | D1 | D2 | D3 | D4 | D5 | D6 |
| Control | 0.0±0.0 | 0.1±0.6 | -1.1±0.8 | 0.8±1.0 | 0.5±1.1 | -0.5±2.0 | -1.2±1.9 |
| Vehicle | 0.0±0.0 | 0.4±0.9 | -0.3±1.3 | -0.9±1.6 | -1.0±1.8 | -1.2±2.0 | -0.9±2.2 |
| Semaglutide | 0.0±0.0 | -5.3±1.0 | -7.2±1.2 | -8.2±1.6 | -7.4±2.8 | -6.7±3.7 | -8.2±3.8 |
| HEC-G115 | 0.0±0.0 | -5.5±0.7 | -8.4±1.0 | -9.7±1.1 | -11.5±1.1 | -12.7±1.7 | -13.4±2.0 |
| HEC-G124 | 0.0±0.0 | -6.0±1.0 | -8.9±1.2 | -9.3±2.1 | -8.8±3.0 | -8.3±2.8 | -8.4±2.6 |

| Group | Weight growth rate (%) | | | | | | |
|---|---|---|---|---|---|---|---|
| | D7 | D10 | D13 | D16 | D19 | D22 | D25 |
| Control | 0.0±1.2 | 0.0±2.8 | 1.7±2.8 | 0.6±3.2 | 2.2±4.0 | 1.5±2.9 | 2.8±3.1 |
| Vehicle | -0.9±2.3 | -2.1±2.6 | -2.2±2.8 | -0.3±2.5 | 0.2±3.0 | 2.8±3.3 | 1.3±4.0 |
| Semaglutide | -8.1±4.7 | -17.6±5.1 | -21.3±5.8 | -22.3±5.3 | -21.6±5.6 | -24.2±6.2 | -23.2±6.0^{a} |
| HEC-G115 | -13.4±2.6 | -22.2±6.2 | -24.3±8.5 | -24.2±7.8 | -23.9±7.7 | -22.4±6.8 | -20.4±6.8^{a} |
| HEC-G124 | -7.1±2.9 | -15.5±2.8 | -18.8±3.9 | -19.1±3.5 | -19.9±4.4 | -20.0±3.5 | -19.2±5.3^{a} |

| | | | | | | | |
|---|---|---|---|---|---|---|---|
| Note: The lowercase letter a of the shoulder mark in the same column indicate significant differences (P<0.05) | | | | | | | |

**Table 10: Effect of long-term administration of HEC-G115 and HEC-G124 on the food intake of DIO mice**

| Group | Cumulative food intake (g/pcs) | | | | | | | | | | | |
|---|---|---|---|---|---|---|---|---|---|---|---|---|
| | D0 | D1 | | D2 | | D3 | | D4 | | D5 | | D6 |
| Control | 3.5 | 7.3 | | 11.5 | | 15.5 | | 19.7 | | 23.1 | | 27.5 |
| Vehicle | 2.0 | 4.3 | | 6.5 | | 8.8 | | 11.5 | | 13.8 | | 16.2 |
| Semaglutide | 0.7 | 1.8 | | 3.2 | | 5.3 | | 8.2 | | 10.2 | | 12.5 |
| HEC-G115 | 0.6 | 1.5 | | 2.6 | | 3.8 | | 5.7 | | 7.4 | | 9.3 |
| HEC-G124 | 0.4 | 1.2 | | 2.6 | | 4.8 | | 7.2 | | 8.7 | | 11.4 |

| Group | Cumulative food intake (g/pcs) | | | | | | | | | | | |
|---|---|---|---|---|---|---|---|---|---|---|---|---|
| | D7-10 | | D11-13 | | D14-16 | | D17-19 | | D20-22 | | D23-27 | |
| Control | 38.8 | | 51.3 | | 62.8 | | 75.1 | | 86.1 | | 103.5^{a} | |
| Vehicle | 22.9 | | 30.0 | | 38.5 | | 46.2 | | 54.2 | | 67.1 | |
| Semaglutide | 15.7 | | 20.7 | | 27.9 | | 35.7 | | 41.8 | | 55.0^{a} | |
| HEC-G115 | 13.3 | | 19.2 | | 25.7 | | 33.7 | | 42.2 | | 55.5^{a} | |
| HEC-G124 | 14.3 | | 19.7 | | 25.0 | | 31.3 | | 37.6 | | 47.6^{a} | |

| | | | | | | | | | | | | |
|---|---|---|---|---|---|---|---|---|---|---|---|---|
| Note: The lowercase letter a of the shoulder mark in the same column indicate significant differences (P<0.05) | | | | | | | | | | | | |

**Table 11: Effect of long-term administration of HEC-G115 and HEC-G124 on body weight and liver index in DIO mice**

| Group | Body weight/g | Liver/g | Liver index/% |
|---|---|---|---|
| Control | 28.1±2.4^{a} | 1.1±0.1^{a} | 3.8±0.0^{a} |
| Vehicle | 46.5±5.2 | 1.9±0.4 | 4.1±0.0 |
| Semaglutide | 33.0±2.7^{a} | 1.0±0.2^{a} | 3.0±0.0^{a} |
| HEC-G115 | 32.4±2.1^{a} | 1.0±0.1^{a} | 3.0±0.0^{a} |
| HEC-G124 | 32.8±2.9^{a} | 0.9±0.1^{a} | 2.8±0.0^{a} |

| | | | |
|---|---|---|---|
| Note: The lowercase letters of the shoulder mark in the same column indicate significant differences (P<0.05) | | | |

**Table 12: Effect of long-term administration of HEC-G115 and HEC-G124 on liver function and blood lipids in DIO mice**

| Group | ALT (U/L) | AST (U/L) | TRIGL(mmo l/L) | CHOL(mmol/ L) |
|---|---|---|---|---|
| Control | 31.9±6.4^{a} | 127.6±31.9 | 1.0±0.2 | 2.2±1.1^{a} |
| Vehicle | 126.4±12.5 | 204.5±78.8 | 1.0±0.1 | 5.4±0.7 |
| Semaglutide | 19.3±4.3^{a} | 109.4±9.2^{a} | 0.7±0.1 | 2.7±0.4^{a} |
| HEC-G115 | 20.7±3.1^{a} | 107.3±11.9^{a} | 0.8±0.2 | 2.9±0.3^{a} |
| HEC-G124 | 28.3±5.8^{a} | 115.3±22.6^{a} | 1.0±0.2 | 2.6±0.3^{a} |

| | | | | |
|---|---|---|---|---|
| Note: The lowercase letters of the shoulder mark in the same column indicate significant differences (P<0.05) Results: The weight loss and feeding inhibition effects of HEC-G115 andHEC-G124 administered once every three days after 4 weeks were comparable to those of the control group Semaglutide administered once a day at the same dose. HEC-G115 and HEC-G124 also significantly improved the liver function and blood lipids of DIO mice, and the effect was similar to that of Semaglutide. | | | | |

Reference throughout this specification to "an embodiment," "some embodiments," "an example," "a specific example," or "some examples," means that a particular feature, structure, material, or characteristic described in connection with the embodiment or example is included in at least one embodiment or example of the present disclosure. In this specification, the schematic expressions of the above terms are not necessarily directed to the same embodiment or example. Furthermore, the particular features, structures, materials, or characteristics may be combined in any suitable manner in one or more embodiments or examples. In addition, those skilled in the art can integrate and combine different embodiments, examples or the features of them as long as they are not contradictory to one another.

Although embodiments of the present invention have been shown and described, it would be appreciated by those skilled in the art that the above embodiments cannot be construed to limit the present disclosure, and changes, alternatives, and modifications can be made in the embodiments without departing from spirit, principles and scope of the present disclosure.

## Claims

1. A GLP-1/GIP dual-target polypeptide comprising a first polypeptide, wherein the first polypeptide has the following amino acid sequence:
X₁X₂X₃GTFX₄SDYSX₅X₆X₇X₈X₉X₁₀X₁₁X₁₂X₁₃ X₁₄FX₁₅X₁₆W LX₁₇X₁₈X₁₉, wherein, X₁ is Y or H, X₂ is A, G or S, X₃ is E or Q, X₄ is I or T, X5 is I or K, X₆ is A, Y, L or I, X₇ is M or L, X₈ is D or E, X₉ is K or E, X₁₀ is I, Q, K, E or L, X₁₁ is H, A or R, X₁₂ is A, Q or V, X₁₃ is K, Q, R or H, X₁₄ is D, E, A or L, X₁₅ is V or I, X₁₆ is N, E, D or Q, X₁₇ is L, I, K or V, X₁₈ is A, E or K, X₁₉ is Q or G;
X₁~X₁₉ do not simultaneously satisfy that X₁ is Y, X₂ is A, X₃ is E, X₄ is I, X₅ is I, X₆ is A, X₇ is M, X₈ is D, X₉ is K, X₁₀ is I, X₁₁ is H, X₁₂ is Q, X₁₃ is Q, X₁₄ is D, X₁₅ is V, X₁₆ is N, X₁₇ is L, X₁₈ is A, X₁₉ is Q.

2. The GLP-1/GIP dual-target polypeptide of claim 1, **characterized in that** the GLP-1/GIP dual-target polypeptide comprises a first polypeptide having the following amino acid sequence:
X₁X₂EGTFTSDYSIX₆LDKX₁₀AQX₁₃X₁₄FX₁₅X₁₆WLX₁₇AX₁₉, wherein, X₁ is Y or H, X₂ is A, G or S, X₆ is A, Y or L, X₁₀ is I, Q, K or L, X₁₃ is Q or R, X₁₄ is D, E or A, X₁₅ is V or I, X₁₆ is E, D or Q, X₁₇ is L, I or K, X₁₉ is Q or G;
wherein, the first polypeptide does not comprise the amino acid sequence YAEGTFISDYSIAMDKIHQQDFVNWLLAQ (SEQ IDNO:122).

3. The GLP-1/GIP dual-target polypeptide of claim 1 or 2, **characterized in that** the GLP-1/GIP dual-target polypeptide further comprises a second polypeptide having the amino acid sequence shown in SEQ ID NO:1.

4. The GLP-1/GIP dual-target polypeptide of any one of claims 1 to 3, **characterized in that** the C-terminus of the first polypeptide is connected with the N-terminus of the second polypeptide.

5. The GLP-1/GIP dual-target polypeptide of any one of claims 1 to 4, **characterized in that** the GLP-1/GIP dual-target polypeptide has at least one of the following amino acid sequences: SEQ ID NO:2-112, SEQ ID NO:117-121.

6. The GLP-1/GIP dual-target polypeptide of claim 5, **characterized in that** the GLP-1/GIP dual-target polypeptide has the amino acid sequence shown below: SEQ ID NO:2, SEQ ID NO:13, SEQ ID NO:16, SEQ ID NO:25-28, SEQ ID NO:37, SEQ ID NO:50-51, SEQ ID NO:56, SEQ ID NO:58, SEQ ID NO:83, SEQ ID NO:85, SEQ ID NO:93, SEQ ID NO:95, SEQ ID NO:101-107, SEQ ID NO:110-112.

7. A fusion protein comprising:
(1) a GLP-1/GIP dual-target polypeptide of any one of claims 1 to 6; and
(2) an Fc fragment, the C-terminus of the GLP-1/GIP dual-target polypeptide is connected with the N-terminus of the Fc fragment.

8. The fusion protein of claim 7, **characterized in that** the Fc fragment is an Fc fragment of a human IgG4 or variant thereof.

9. The fusion protein of claim 7 or 8, **characterized in that** the Fc fragment comprises an amino acid sequence shown in SEQ ID NO: 113.

10. The fusion protein of any one of claims 7 to 9, **characterized in that** the fusion protein further comprises a linker peptide with an amino acid sequence shown in SEQ ID NO: 114.

11. The fusion protein of any one of claims 7 to 10, **characterized in that** the N-terminus of the linker peptide is connected with the C-terminus of the GLP-1/GIP dual-target polypeptide, and the C-terminus of the linker peptide is connected with the N-terminus of the Fc fragment.

12. A nucleic acid molecule encoding the GLP-1/GIP dual-target polypeptide of any one of claims 1 to 6 or the fusion protein of any one of claims 7 to 11.

13. An expression vector comprising the nucleic acid molecule of claim 12.

14. The expression vector of claim 13, **characterized in that** the expression vector is a eukaryotic expression vector.

15. A recombinant cell carrying the nucleic acid molecule of claim 12 or the expression vector of claim 13 or 14.

16. A pharmaceutical composition comprising the GLP-1/GIP dual-target polypeptide of any one of claims 1 to 6, the fusion protein of any one of claims 7 to 11, the nucleic acid molecule of claim 12, the expression vector of claim 13 or 14, or the recombinant cell of claim 15.

17. Use of the GLP-1/GIP dual-target polypeptide of any one of claims 1 to 6, the fusion protein of any one of claims 7 to 11, the nucleic acid molecule of claim 12, the expression vector of claim 13 or 14, the recombinant cell of claim 15 in the manufacture of a medicament for treating diabetes, obesity, fatty liver disease, non-alcoholic fatty liver disease, and non-alcoholic steatohepatitis, dyslipidemia and metabolic syndrome.

18. A method for preparing the fusion protein of any one of claims 7 to 11 comprising:
1) Constructing the expression vector of claim 12 or 13;
2) Introducing the expression vector into host cells to obtain recombinant cells to express the fusion protein.
